# EUROPEAN PATENT SPECIFICATION

(11) **EP 1 788 861 B1**
(45) Date of publication and mention of the grant of the patent: **12.04.2017**
(21) Application number: 05808646.3
(22) Date of filing: 24.08.2005
(51) Int. Cl.: C12N 15/82, A01H 5/00

(54) **ADENYLATE TRANSLOCATOR PROTEIN GENE NON-CODING REGULATORY ELEMENTS FOR USE IN PLANTS**
NICHTKODIERENDE REGULATIONSELEMENTE DES ADENYLATTRANSLOKATORPROTEINGENS ZUR VERWENDUNG BEI PFLANZEN
ELEMENTS REGULATEURS NON CODANTS DE GENE DE PROTEINE DE TRANSLOCATEUR D'ADENYLATE UTILISES DANS DES VEGETAUX

(30) Priority: 24.08.2004 US 604127 P
(43) Date of publication of application: 30.05.2007
(73) Proprietor: Monsanto Technology, LLC, St. Louis, MO 63167 (US)
(72) Inventor: FLASINSKI, Stanislaw, Chesterfield, MO 63017 (US); CHEN, Yun-Chia, Sophia, Chicago, IL 60611 (US)
(74) Representative: dompatent von Kreisler Selting Werner - Partnerschaft von Patent- und Rechtsanwälten mbB
(86) International application number: PCT/US2005/029831
(87) International publication number: WO 2006/023869

(56) References cited:
- WO-A-01/20009
- US-A1- 2001 047 523
- DATABASE GENBANK [Online] 19 January 2001 LIN X. ET AL.: 'Arabidopsis thailiana chromosome 3 BAC F17014 genomic sequence', XP003002386 Retrieved from NCBI Database accession no. (AC012562)
- HU T. ET AL.: 'Agrobacterium-mediated Large-scale transformation of wheat (Triticum aestivum L.) using glyphosate selection' PLANT CELL REPORTS vol. 21, no. 10, June 2003, pages 1010 - 1019, XP003002387

<110> Yun-Chia Sophia Chen Flasinski, Stanislaw
<120> ADENYLATE TRANSLOCATOR PROTEIN 5' UNTRANSLATED MOLECULES FOR USE IN PLANTS
<130> 38-21(53713)B
<150> US 60/604,127
   <151> 2004-08-24
<160> 6
<210> 1
   <211> 1705
   <212> DNA
   <213> Arabidopsis thaliana
<400> 1
   ttgtggcccc gagtgactat gagacaagtc tgatcgaagt cgtcgagaag tgatgattct   60
   tgttgttgta gttcttttta aaagtcccac ctgagcctct atagactctg attctctttt  120
   aagttactat tttcaccgct ctctaataag gctcgtgatt ttttgggagc catactgtat  180
   actcgccggc cctctcacga tgttgttcaa ttcacagact aaatttgatt tatctatttc  240
   gccaaaacat aacttcatta aaaaatgttc tccaaataac taaacgaatt aaataaaaga  300
   aacctttcat gtaaagttaa aggtatgaga ctttaagggc agttgctgaa cattcgtaac  360
   acatgggaga acaatagaga aagttgaaaa gaaacgtagc atatagaaaa attatctttg  420
   taaccaagtt gatttagaaa aatatcacta tttgtgaaaa atactagatc agtttattat  480
   tacttttttt ttttttgtat attcacaaat atcatattca tatagaagaa aataaacaaa  540
   gttgtaaaaa tctggcattt aaaataaaat tgaacacttc aatttatttc ctttcataat  600
   aataattttg gcataagata tttgcaaatt gatctggttc ggtatggtcg acaaaataat  660
   tttccacgct acccttccag ccgtccattc actatttgcc ctcaacgtta ccaaataacg  720
   gtccagattc ctagggcaag atctaacggt tagcaagtaa agtcgtacca tcagaaagaa  780
   taacaattct ttcacaaagt aaacataacc aacggttaac aagttcttag ggttaaatca  840
   gtaagatcca acggatatta aattgcaagg cccaaatagt ttttttgcag cagataataa  900
   ctcgtcccca ctggcgagtg acgaccgaga ctctgtgacc ctatttttcg agacgataaa  960
   agggcaaaca atcgctcttt tcaaagctcg cctcttcacc acagagaaaa cttcgtctct 1020
   cttctctgct tcgccctctc atttcctgtg agataaaggc ggagtctctc tccagttatt 1080
   ttgctcatcc atcgattctt aggtatgact cgtttctctc agatctgtga ttctttataa 1140
   tctcgtcgtt cttcaaatca ttgttatatt cgtttcttcg atctgtgttt tttagatctg 1200
   taaggtaaat gagacgtttc gatctgtaga tctgattgtt atattgatag attatgttat 1260
   ctgctttgct taaagtccga tcggaatgtt ttgtgctcat tgtcgaatat ctgatgtatc 1320
   ggtttcatag atctgcttct ttttgtgcgt ttcgttgatc tgataatctt ctagtgatca 1380
   aaatcgtttg gatctgttga ctttagttta aaatgtatcc gatctgatgt cgaggcttca 1440
   ttattggaag ttgttattgt tgtaatcctg atttaagttg ctgttcttaa atttatatga 1500
   tctttgcgtt ataatatgac atggtagatc ttggttcatg gttcactgtt ttccaataaa 1560
   cttggtttgt ttggttggat agcgttctgt gatacgacca tgtcttgtgt tggataagaa 1620
   ttctctgaat ttccttggct ggtttgtagt atgttattca cgtctggttt ctcatcaatg 1680
   attatgtgat tttgcagagt tcacc                                      1705
<210> 2
   <211> 990
   <212> DNA
   <213> Arabidopsis thaliana
<400> 2
   ttgtggcccc gagtgactat gagacaagtc tgatcgaagt cgtcgagaag tgatgattct   60
   tgttgttgta gttcttttta aaagtcccac ctgagcctct atagactctg attctctttt  120
   aagttactat tttcaccgct ctctaataag gctcgtgatt ttttgggagc catactgtat  180
   actcgccggc cctctcacga tgttgttcaa ttcacagact aaatttgatt tatctatttc  240
   gccaaaacat aacttcatta aaaaatgttc tccaaataac taaacgaatt aaataaaaga  300
   aacctttcat gtaaagttaa aggtatgaga ctttaagggc agttgctgaa cattcgtaac  360
   acatgggaga acaatagaga aagttgaaaa gaaacgtagc atatagaaaa attatctttg  420
   taaccaagtt gatttagaaa aatatcacta tttgtgaaaa atactagatc agtttattat  480
   tacttttttt ttttttgtat attcacaaat atcatattca tatagaagaa aataaacaaa  540
   gttgtaaaaa tctggcattt aaaataaaat tgaacacttc aatttatttc ctttcataat  600
   aataattttg gcataagata tttgcaaatt gatctggttc ggtatggtcg acaaaataat  660
   tttccacgct acccttccag ccgtccattc actatttgcc ctcaacgtta ccaaataacg  720
   gtccagattc ctagggcaag atctaacggt tagcaagtaa agtcgtacca tcagaaagaa  780
   taacaattct ttcacaaagt aaacataacc aacggttaac aagttcttag ggttaaatca  840
   gtaagatcca acggatatta aattgcaagg cccaaatagt ttttttgcag cagataataa  900
   ctcgtcccca ctggcgagtg acgaccgaga ctctgtgacc ctatttttcg agacgataaa  960
   agggcaaaca atcgctcttt tcaaagctcg                                   990
<210> 3
   <211> 715
   <212> DNA
   <213> Arabidopsis thaliana
<400> 3
   cctcttcacc acagagaaaa cttcgtctct cttctctgct tcgccctctc atttcctgtg   60
   agataaaggc ggagtctctc tccagttatt ttgctcatcc atcgattctt aggtatgact  120
   cgtttctctc agatctgtga ttctttataa tctcgtcgtt cttcaaatca ttgttatatt  180
   cgtttcttcg atctgtgttt tttagatctg taaggtaaat gagacgtttc gatctgtaga  240
   tctgattgtt atattgatag attatgttat ctgctttgct taaagtccga tcggaatgtt  300
   ttgtgctcat tgtcgaatat ctgatgtatc ggtttcatag atctgcttct ttttgtgcgt   360
   ttcgttgatc tgataatctt ctagtgatca aaatcgtttg gatctgttga ctttagttta  420
   aaatgtatcc gatctgatgt cgaggcttca ttattggaag ttgttattgt tgtaatcctg  480
   atttaagttg ctgttcttaa atttatatga tctttgcgtt ataatatgac atggtagatc  540
   ttggttcatg gttcactgtt ttccaataaa cttggtttgt ttggttggat agcgttctgt  600
   gatacgacca tgtcttgtgt tggataagaa ttctctgaat ttccttggct ggtttgtagt  660
   atgttattca cgtctggttt ctcatcaatg attatgtgat tttgcagagt tcacc        715
<210> 4
   <211> 595
   <212> DNA
   <213> Arabidopsis thaliana
<400> 4
   gtatgactcg tttctctcag atctgtgatt ctttataatc tcgtcgttct tcaaatcatt   60
   gttatattcg tttcttcgat ctgtgttttt tagatctgta aggtaaatga gacgtttcga  120
   tctgtagatc tgattgttat attgatagat tatgttatct gctttgctta aagtccgatc  180
   ggaatgtttt gtgctcattg tcgaatatct gatgtatcgg tttcatagat ctgcttcttt  240
   ttgtgcgttt cgttgatctg ataatcttct agtgatcaaa atcgtttgga tctgttgact  300
   ttagtttaaa atgtatccga tctgatgtcg aggcttcatt attggaagtt gttattgttg  360
   taatcctgat ttaagttgct gttcttaaat ttatatgatc tttgcgttat aatatgacat  420
   ggtagatctt ggttcatggt tcactgtttt ccaataaact tggtttgttt ggttggatag  480
   cgttctgtga tacgaccatg tcttgtgttg gataagaatt ctctgaattt ccttggctgg  540
   tttgtagtat gttattcacg tctggtttct catcaatgat tatgtgattt tgcag        595
<210> 5
   <211> 30
   <212> DNA
   <213> Arabidopsis thaliana
<400> 5
   tccaaagctt tgtggccccg agtgactatg 30
<210> 6
   <211> 27
   <212> DNA
   <213> Arabidopsis thaliana
<400> 6
   atcaaccatg gtgaactctg caaaatc 27

## Description

### FIELD OF THE INVENTION

The invention relates to the field of plant molecular biology and plant genetic engineering and polynucleotide molecules useful for the expression of transgenes in plants. In particular, the invention relates to DNA molecules useful for the regulation of transgene expression in plants.

### BACKGROUND

One of the goals of plant genetic engineering is to produce plants with agronomically desirable characteristics or traits. The proper expression of a desirable transgene in a transgenic plant is one way to achieve this goal. Regulatory elements such as promoters, leaders, and introns are non-coding polynucleotide molecules that play an integral part in the overall expression of genes in living cells. Isolated regulatory elements that function in plants are therefore useful for modifying plant phenotypes through the methods of genetic engineering.

Many regulatory elements are available and are useful for providing good overall expression of a transgene. For example, constitutive promoters such as P-FMV.35S, the promoter from the 35S transcript of the Figwort mosaic virus (U.S. Patent No. 6,051,753); P-CaMV 35S, the promoter from the 35S RNA transcript of the Cauliflower mosaic virus (U.S. Patent 5,530,196); P-rice Actin 1, the promoter from the actin 1 gene of *Oryza sativa* (U.S. Patent 5,641,876); and P-nos, the promoter from the nopaline synthase gene of *Agrobacterium tumefaciens* are known to provide some level of gene expression in most or all of the tissues of a plant during most or all of the plant's lifespan. While previous work has provided a number of regulatory elements useful to affect gene expression in transgenic plants, there is still a great need for novel regulatory elements with beneficial expression characteristics. In particular, there is a need for regulatory elements that are capable of directing expression of transgenes in transgenic crop plants at high levels and in particular tissues, organs, or during specific developmental stages of plant growth. Many previously identified regulatory elements fail to provide the patterns or levels of expression required to fully realize the benefits of expression of selected genes in transgenic plants.

Adenylate translocator protein is involved in intercellular transport of substrates of the starch biosynthetic pathway. Members of this family occur in many plant species including corn, wheat, rice, barley, soybean, and *Arabidopsis.* We hypothesized that a promoter from an adenylate translocator protein gene would have a constitutive expression pattern and that the promoter and regulatory elements would be useful to direct expression of a transgene such as a glyphosate resistant 5-enolpyruvylshikimate-3-phosphate synthase (EPSPS) transgene to produce a glyphosate tolerant plant or other herbicide tolerance genes where constitutive expression is required. The efficient production of glyphosate tolerant plants requires the use of a promoter and regulatory elements capable of directing transgene expression in all tissues including the glyphosate sensitive reproductive organs such as anthers and meristem tissues. The present invention thus provides such promoters and regulatory elements isolated from the adenylate translocator protein gene of *Arabidopsis thaliana.*

We found that isolated non-coding regulatory elements for the adenylate translocator protein gene, particularly the promoter, leader and intron elements, provided enhanced expression of an operably linked transgene in a transgenic crop plant.

### SUMMARY OF THE INVENTION

The present invention describes the composition and utility for the Adenylate Translocator protein gene non-coding regulatory element molecules, hereinafter referred to as ANT1-NCRE molecules.

The present invention includes and provides polynucleotide molecule having gene regulatory activity consisting of an adenylate translocase gene non-coding regulatory element polynucleotide molecule, that (a) exhibits an 95% or greater identity to the full-length sequence of SEQ ID NO:1, or to a sequence that exhibits complete complementarity thereto, or any fragment of SEQ ID NO:1 comprising at least 95 contiguous nucleotide bases, each thereof capable of directing transgene expression in glyphosate sensitive reproductive organs; or (b) has the sequence of SEQ ID NO:1; wherein said polynucleotide molecule is operably linked to a transcribable polynucleotide molecule that is heterologous to said polynucleotide molecule.

The present invention includes and provides a polynucleotide construct comprising a polynucleotide molecule comprising an adenylate translocase gene non-coding regulatory element polynucleotide molecule, that (a) exhibits an 95% or greater identity to the full-length sequence of SEQ ID NO:1, or to a sequence that exhibits complete complementarity thereto, or any fragment of SEQ ID NO:1 comprising at least 95 contiguous nucleotide bases, each thereof capable of directing transgene expression in glyphosate sensitive reproductive organs; or (b) has the sequence of SEQ ID NO:1, wherein said polynucleotide molecule is operably linked to a transcribable polynucleotide molecule that is heterologous to said polynucleotide molecule. The present invention includes and provides a transgenic plant cell, a transgenic plant or seed thereof all of which having the above polynucleotide construct integrated into its genome.

The present invention includes and provides a method of inhibiting weed growth in a field of transgenic glyphosate tolerant crop plants comprising: (i) planting the transgenic plants transformed with an expression cassette comprising a polynucleotide molecule having gene regulatory activity comprising an adenylate translocase gene non-coding regulatory element polynucleotide molecule, that (a) exhibits an 95% or greater identity to the full-length sequence of SEQ ID NO:1, or to a sequence that exhibits complete complementarity thereto, or any fragment of SEQ ID NO:1 comprising at least 95 contiguous nucleotide bases, each thereof capable of directing transgene expression in glyphosate sensitive reproductive organs; or (b) has the sequence of SEQ ID NO:1, operably linked to a polynucleotide molecule encoding a glyphosate tolerance gene and (ii) applying glyphosate to the field at an application rate that inhibits the growth of weeds, wherein the growth and yield of the transgenic crop plant is not substantially affected by the glyphosate application.

In one embodiment the invention provides an adenylate transiocator (Ant1) protein gene 5' untranslated regulatory elements isolated from *Arabidopsis thaliana,* and exemplary DNA molecules provided as SEQ ID NO: 1 and SEQ ID NO: 2 useful for modulating the expression of transgenes in plants. The transgenic plant preferably expresses an agronomically desirable phenotype, in particular herbicide tolerance, more specifically, tolerance to glyphosate herbicide.

### BRIEF DESCRIPTION OF THE DRAWINGS

Figure 1. illustrates plasmid map of pMON71524
Figure 2. illustrates plasmid map of pMON26140
Figure 3. illustrates plasmid map of pMON20999

### DETAILED DESCRIPTION

The invention disclosed herein provides DNA molecules comprising non-coding regulatory elements from the adenylate translocator protein gene, herein referred to as Ant1. The non-coding regulatory element polynucleotide molecules described herein are henceforth referred to as ANT1-NCRE molecules. The design, construction, and use of the polynucleotide molecules is one object of this invention. The polynucleotide sequences of the polynucleotide molecules is provided as SEQ ID NO:1 and SEQ ID NO: 2. These polynucleotide molecule are capable of affecting the transcription of an operably linked transcribable polynucleotide molecule in both vegetative and reproductive tissues of plants and therefore can selectively regulate expression of transgenes in these tissues. The polynucleotide molecule of SEQ ID NO:1 is a 1.7 kb (kilobase) genomic DNA fragment that further comprises regulatory elements identified within the DNA fragment as a promoter element (SEQ ID NO:2), a leader-intron element (SEQ ID NO:3) and an intron element (SEQ ID NO:4). These elements are useful for providing expression of transgenes in plants.

### Definitions

The following definitions and methods are provided to better define the present invention and to guide those of ordinary skill in the art in the practice of the present invention. Unless otherwise noted, terms are to be understood according to conventional usage by those of ordinary skill in the relevant art.

As used herein, the term "fragment" or "fragment thereof' refers to a finite polynucleotide sequence length that comprises at least 95 contiguous nucleotide bases wherein its complete sequence in entirety is identical to a contiguous component of the referenced polynucleotide molecule.

As used herein, the term "polynucleotide molecule" refers to the single- or double-stranded DNA or RNA molecule of genomic or synthetic origin, i.e., a polymer of deoxyribonucleotide or ribonucleotide bases, respectively, read from the 5' (upstream) end to the 3' (downstream) end. As used herein, the term "polynucleotide sequence" refers to the sequence of a polynucleotide molecule. The nomenclature for nucleotide bases as set forth at 37 CFR § 1.822 is used herein. As used herein, the term "regulatory element" refers to a polynucleotide molecule having gene regulatory activity, i.e. one that has the ability to affect the transcription or translation of an operably linked transcribable polynucleotide molecule. Regulatory elements such as promoters, leaders, introns, and transcription termination regions are non-coding polynucleotide molecules having gene regulatory activity which play an integral part in the overall expression of genes in living cells. Isolated regulatory elements that function in plants are therefore useful for modifying plant phenotypes through the methods of genetic engineering. By "regulatory element" it is intended a series of nucleotides that determines if, when, and at what level a particular gene is expressed. The regulatory DNA sequences specifically interact with regulatory proteins or other proteins.

As used herein, the term "gene regulatory activity" refers to a polynucleotide molecule capable of affecting transcription or translation of an operably linked polynucleotide molecule. An isolated polynucleotide molecule having gene regulatory activity may provide temporal or spatial expression or modulate levels and rates of expression of the operably linked polynucleotide molecule. An isolated polynucleotide molecule having gene regulatory activity may comprise a promoter, intron, leader, or 3' transcriptional termination region.

As used herein, the term "gene expression" or "expression" refers to the transcription of a DNA molecule into a transcribed RNA molecule. Gene expression may be described as related to temporal, spatial, developmental, or morphological qualities as well as quantitative or qualitative indications. The transcribed RNA molecule may be translated to produce a protein molecule or may provide an antisense or other regulatory RNA molecule.

As used herein, an "expression pattern" is any pattern of differential gene expression. In a preferred embodiment, an expression pattern is selected from the group consisting of tissue, temporal, spatial, developmental, stress, environmental, physiological, pathological, cell cycle, and chemically responsive expression patterns.

As used herein, an "enhanced expression pattern" is any expression pattern for which an operably linked nucleic acid sequence is expressed at a level greater than 0.01%; preferably in a range of 0.5% to 20% (w/w) of the total cellular RNA or protein.

As used herein, the term "operably linked" refers to a first polynucleotide molecule, such as a promoter, connected with a second transcribable polynucleotide molecule, such as a gene of interest, where the polynucleotide molecules are so arranged that the first polynucleotide molecule affects the function of the second polynucleotide molecule. The two polynucleotide molecules may or may not be part of a single contiguous polynucleotide molecule and may or may not be adjacent. For example, a promoter is operably linked to a gene of interest if the promoter regulates or mediates transcription of the gene of interest in a cell.

As used herein, the term "transcribable polynucleotide molecule" refers to any polynucleotide molecule capable of being transcribed into a RNA molecule, including protein coding sequences (e.g. transgenes) and non-coding sequences (e.g. a molecule useful for gene suppression). Methods are known in the art for constructing and introducing constructs into a cell in such a manner that the transcribable polynucleotide molecule is transcribed into a functional mRNA molecule that is translated and therefore expressed as a protein product. Methods are known in the art for constructing and introducing constructs into a cell in such a manner that the transcribable polynucleotide molecule is transcribed into a molecule that is capable of causing gene suppression. For example, posttranscriptional gene suppression using a construct with an anti-sense oriented transcribable polynucleotide molecule to regulate gene expression in plant cells is disclosed in U.S. Patent Nos. 5,107,065 and 5,759,829; posttranscriptional gene suppression using a construct with a sense-oriented transcribable polynucleotide molecule to regulate gene expression in plants is disclosed in U.S. Patent Nos. 5,283,184 and 5,231,020. For the practice of the present invention, conventional compositions and methods for preparing and using constructs and host cells are well known to one skilled in the art, see for example, JF Sambrook, DW Russell, and N Irwin. (2000) Molecular Cloning: A Laboratory Manual, 3rd edition Volumes 1, 2, and 3. Cold Spring Harbor Laboratory Press, hereafter referred to as Sambrook et al., 2000. Constructs may also be constructed to be capable of expressing antisense RNA molecules, in order to inhibit translation of a specific RNA molecule of interest. For the practice of the present invention, conventional compositions and methods for preparing and using constructs and host cells are well known to one skilled in the art, see for example, Molecular Cloning: A Laboratory Manual, 3rd edition Volumes 1, 2, and 3 (2000) J.F. Sambrook, D.W. Russell, and N. Irwin, Cold Spring Harbor Laboratory Press.

### Promoters

The composition and utility for the Adenylate Translocator protein gene non-coding regulatory element molecules, hereinafter referred to as ANT1-NCRE molecules are described herein. These ANT1-NCRE molecules include promoters. As used herein, the term "promoter" refers to a polynucleotide molecule that is involved in recognition and binding of RNA polymerase II and other proteins such as transcription factors (trans-acting protein factors that regulate transcription) to initiate transcription of an operably linked gene. Promoters may themselves contain sub-elements such as cis-elements or enhancer domains that effect the transcription of operably linked genes. A "plant promoter" is a native or non-native promoter that is functional in plant cells. A plant promoter can be used as a 5' regulatory element for modulating expression of an operably linked gene or genes. Plant promoters may be defined by their temporal, spatial, or developmental expression pattern.

Any of the nucleic acid molecules described herein may comprise nucleic acid sequences comprising promoters. Promoters of the present invention can include between 300 bp upstream and 10 kb upstream of the trinucleotide ATG sequence at the start site of a protein coding region. Promoters of the present invention can preferably include between 300 bp upstream and 5 kb upstream of the trinucleotide ATG sequence at the start site of a protein coding region. Promoters of the present invention can more preferably include between 300 bp upstream and 2 kb upstream of the trinucleotide ATG sequence at the start site of a protein coding region. Promoters of the present invention can include between 300 bp upstream and 1 kb upstream of the trinucleotide ATG sequence at the start site of a protein coding region. While in many circumstances a 300 bp promoter may be sufficient for expression, additional sequences may act to further regulate expression, for example, in response to biochemical, developmental or environmental signals.

### Promoter Activity

The activity or strength of a promoter may be measured in terms of the amount of mRNA or protein accumulation it specifically produces, relative to the total amount of mRNA or protein. The promoter preferably expresses an operably linked nucleic acid sequence at a level greater than 0.01%; preferably in a range of 0.5% to 20% (w/w) of the total cellular RNA or protein. Alternatively, the activity or strength of a promoter may be expressed relative to a well-characterized promoter (for which transcriptional activity was previously assessed). For example, a less-characterized promoter may be operably linked to a reporter sequence (e.g., GUS) and introduced into a specific cell type. A well-characterized promoter (e.g. the 35S promoter) is similarly prepared and introduced into the same cellular context. Transcriptional activity of the unknown promoter is determined by comparing the amount of reporter expression, relative to the well characterized promoter. In one embodiment, the activity of the present promoter is as strong as the 35S promoter when compared in the same cellular context. The cellular context is preferably maize, sorghum, corn, barley, wheat, canola, soybean, or maize; and more preferably is maize, sorghum, corn, barley, or wheat; and most preferably is maize.

### Cis Elements

Promoters may contain one or more of the following elements: a CAAT, a GC, or a TATA cis element. Moreover, the promoters of the present invention may contain one or more cis elements in addition to a GC, CAAT and/or a TATA box.

Many regulatory elements act in cis fashion ("cis elements") and are believed to affect DNA topology, producing local conformations that selectively allow or restrict access of RNA polymerase to the DNA template or that facilitate selective opening of the double helix at the site of transcriptional initiation. Cis elements occur within promoters, and promoter modulating sequences (inducible elements). Cis elements can be identified using known cis elements as a target sequence or target motif in the BLAST programs of the present invention.

Promoters of the present invention may include homologues of cis elements known to effect gene regulation and that show homology with the promoter sequences of the present invention.

### 5' Non-Translated Leader and Enhancer Sequences

The composition and utility for the Adenylate Translocator protein gene non-coding regulatory element molecules, hereinafter referred to as ANT1-NCRE molecules are described herein. These ANT1-NCRE molecules include 5' non-translated leader sequences.

As used herein, the term "leader" refers to a non-coding polynucleotide molecule. A leader may be isolated from the untranslated 5' region (5' UTR) of a genomic copy of a gene and defined generally as a segment between the transcription start site (TSS) and the coding sequence start site. Alternately, leaders may be synthetically produced or manipulated non-coding DNA elements. A "plant leader" is a native or non-native leader that is functional in plant cells. A plant leader can be used as a 5' regulatory element for modulating expression of an operably linked gene or genes.

As used herein, the term "enhancer domain" refers to a cis-acting transcriptional regulatory element, a.k.a. cis-element, which confers an aspect of the overall control of gene expression. An enhancer domain may function to bind transcription factors. Some enhancer domains bind more than one transcription factor, and transcription factors may interact with different affinities with more than one enhancer domain. Enhancer domains can be identified by a number of techniques, including deletion analysis, *i.e.,* deleting one or more nucleotides from the 5' end or internal to a promoter; DNA binding protein analysis using DNase I footprinting, methylation interference, electrophoresis mobility-shift assays, *in vivo* genomic footprinting by ligation-mediated PCR, and other conventional assays; or by DNA sequence similarity analysis with known cis-element motifs by conventional DNA sequence comparison methods. The fine structure of an enhancer domain can be further studied by mutagenesis (or substitution) of one or more nucleotides or by other conventional methods. Enhancer domains can be obtained by chemical synthesis or by isolation from promoters that include such elements, and they can be synthesized with additional flanking nucleotides that contain useful restriction enzyme sites to facilitate subsequence manipulation.

Translational enhancers may also be incorporated as part of the recombinant vector. Thus the recombinant vector may preferably contain one or more 5' non-translated leader sequences which serve to enhance expression of the nucleic acid sequence. Such enhancer sequences may be desirable to increase or alter the translational efficiency of the resultant mRNA. Examples of other non-coding regulatory element 5' nucleic acid leader sequences include dSSU 5', PetHSP70 5', and GmHSP17.9 5'.

### Introns

The composition and utility for the Adenylate Translocator protein gene non-coding regulatory element molecules, hereinafter referred to as ANT1-NCRE molecules are described herein. These ANT I-NCRE molecules include introns. As used herein, the term "intron" refers to a non-coding polynucleotide molecule. Introns may be isolated from the intervening (non-coding) sequence of a genomic copy of a gene and may be defined generally as a region spliced out during mRNA processing prior to translation. Alternately, introns may be synthetically produced or manipulated non-coding DNA elements. Introns may themselves contain sub-elements such as cis-elements or enhancer domains that effect the transcription of operably linked genes. A "plant intron" is a native or non-native intron that is functional in plant cells. A plant intron can be used as a regulatory element for modulating expression of an operably linked gene or genes.

The transcribable polynucleotide molecule sequence in the recombinant vector may comprise introns. The introns may be heterologous with respect to the transcribable polynucleotide molecule sequence. Examples of other non-coding regulatory element introns include the corn actin intron and the corn HSP70 intron.

### Transcribable Polynucleotide Molecules

The ANT1-NCRE molecules may be operably linked to a transcribable polynucleotide molecule sequence that is heterologous with respect to the ANT1-NCRE molecules.

The term "heterologous" refers to the relationship between two or more nucleic acid or protein sequences that are derived from different sources. For example, a promoter is heterologous with respect to a transcribable polynucleotide sequence if such a combination is not normally found in nature. In addition, a particular sequence may be "heterologous" with respect to a cell or organism into which it is inserted (i.e. does not naturally occur in that particular cell or organism).

The transcribable polynucleotide molecule sequence may generally be any nucleic acid sequence for which an increased level of transcription is desired. The transcribable polynucleotide molecule sequence preferably encodes a polypeptide that is suitable for incorporation into the diet of a human or an animal. Suitable transcribable polynucleotide molecule sequence include those encoding a yield protein, a stress resistance protein, a developmental control protein, a tissue differentiation protein, a meristem protein, an environmentally responsive protein, a senescence protein, a hormone responsive protein, an abscission protein, a source protein, a sink protein, a flower control protein, a seed protein, an herbicide resistance protein, a disease resistance protein, a fatty acid biosynthetic enzyme, a tocopherol biosynthetic enzyme, an amino acid biosynthetic enzyme, and an insecticidal protein.

Alternatively, the ANT1-NCRE molecules and transcribable polynucleotide molecule sequence may be designed to down-regulate a specific nucleic acid sequence. This is typically accomplished by linking the ANT1-NCRE molecules to a transcribable polynucleotide molecule sequence that is oriented in the antisense direction. One of ordinary skill in the art is familiar with such antisense technology. Briefly, as the antisense nucleic acid sequence is transcribed, it hybridizes to and sequesters a complementary nucleic acid sequence inside the cell. This duplex

RNA molecule cannot be translated into a protein by the cell's translational machinery. Any nucleic acid sequence may be negatively regulated in this manner.

### Modified Transcribable Polynucleotide Molecule Sequences

The ANT1-NCRE molecules may also be operably linked to a modified transcribable polynucleotide molecule sequence that is heterologous with respect to the ANT1-NCRE molecules. The transcribable polynucleotide molecule sequence may be modified to provide various desirable features. For example, a transcribable polynucleotide molecule sequence may be modified to increase the content of essential amino acids, enhance translation of the amino acid sequence, alter post-translational modifications *(e.g*., phosphorylation sites), transport a translated product to a compartment inside or outside of the cell, improve protein stability, insert or delete cell signaling motifs.

### Polynucleotide Molecule Isolation and Modification Methods

The present invention includes a polynucleotide molecule having a nucleic acid sequence that: exhibits an 95% or greater identity to the full-length sequence of SEQ ID NO:1, or to a sequence that exhibits complete complementarity thereto, or any fragment of SEQ ID NO:1 comprising at least 95 contiguous nucleotide bases, each thereof capable of directing transgene expression in glyphosate sensitive reproductive organs. The present invention also provides a nucleic acid molecule comprising a nucleic acid sequence of SEQ ID NO:1 and SEQ ID NO:2.

As used herein, an "isolated polynucleotide molecule" refers to a RNA or DNA molecule that is at least partially separated from other molecules normally associated with it in its native state. In one embodiment, the term "isolated" is also used herein in reference to a polynucleotide molecule that is at least partially separated from nucleic acids which normally flank the polynucleotide in its native state. Thus, polynucleotides fused to regulatory or coding sequences with which they are not normally associated, for example as the result of recombinant techniques, are considered isolated herein. Such molecules are considered isolated even when present, for example in the chromosome of a host cell, or in a nucleic acid solution. The term "isolated" as used herein is not intended to encompass molecules present in their native state. An isolated polynucleotide is an "isolated" molecule if it occurs as a component of a transgene in a transgenic plant. The use of the polynucleotide of the present invention in a transgenic plant is an object of the present invention.

The term "hybridization" refers generally to the ability of nucleic acid molecules to join via complementary base strand pairing. Such hybridization may occur when nucleic acid molecules are contacted under appropriate conditions (see also, "specific hybridization," below). "Specifically hybridizes" refers to the ability of two nucleic acid molecules to form an antiparallel, double-stranded nucleic acid structure. A nucleic acid molecule is said to be the "complement" of another nucleic acid molecule if they exhibit "complete complementarity," i.e., each nucleotide in one sequence is complementary to its base pairing partner nucleotide in another sequence. Two molecules are said to be "minimally complementary" if they can hybridize to one another with sufficient stability to permit them to remain annealed to one another under at least conventional "low-stringency" conditions. Similarly, the molecules are said to be "complementary" if they can hybridize to one another with sufficient stability to permit them to remain annealed to one another under conventional "high-stringency" conditions. Nucleic acid molecules that hybridize to other nucleic acid molecules, e.g., at least under low stringency conditions are said to be "hybridizable cognates" of the other nucleic acid molecules. Conventional low stringency and high stringency conditions are described herein and by Sambrook et al., Molecular Cloning, A Laboratory Manual, 2nd Ed., Cold Spring Harbor Press, Cold Spring Harbor, New York (1989) and by Haymes et al., Nucleic Acid Hybridization, A Practical Approach, IRL Press, Washington, DC (1985). Departures from complete complementarity are permissible, as long as such departures do not completely preclude the capacity of the molecules to form a double-stranded structure.

As used herein "sequence identity" refers to the extent to which two optimally aligned polynucleotide or peptide sequences are invariant throughout a window of alignment of components, e.g., nucleotides or amino acids. An "identity fraction" for aligned segments of a test sequence and a reference sequence is the number of identical components which are shared by the two aligned sequences divided by the total number of components in reference sequence segment, i.e., the entire reference sequence or a smaller defined part of the reference sequence. As used herein, the term "percent sequence identity" or "percent identity" refers to the percentage of identical nucleotides in a linear polynucleotide sequence of a reference polynucleotide molecule (or its complementary strand) as compared to a test polynucleotide molecule (or its complementary strand) when the two sequences are optimally aligned (with appropriate nucleotide insertions, deletions, or gaps totaling less than 20 percent of the reference sequence over the window of comparison). Optimal alignment of sequences for aligning a comparison window are well known to those skilled in the art and may be conducted by tools such as the local homology algorithm of Smith and Waterman, the homology alignment algorithm of Needleman and Wunsch, the search for similarity method of Pearson and Lipman, and preferably by computerized implementations of these algorithms such as GAP, BESTFIT, FASTA, and TFASTA available as part of the GCG^{®} Wisconsin Package^{®} (Accelrys Inc., San Diego, CA). An "identity fraction" for aligned segments of a test sequence and a reference sequence is the number of identical components which are shared by the two aligned sequences divided by the total number of components in the reference sequence segment, *i.e.,* the entire reference sequence or a smaller defined part of the reference sequence. Percent sequence identity is represented as the identity fraction times 100. The comparison of one or more polynucleotide sequences refer to a full-length polynucleotide sequence.

As used herein, the term "substantial percent sequence identity" refers to a percent sequence identity of at least about 95% or about 98% or about 99% sequence identity. Thus, one embodiment of the invention is a polynucleotide molecule that has 95% or 98% or 99% sequence identity with a polynucleotide sequence described herein.

Polynucleotide molecules that are capable of regulating or affecting the transcription of an operably linked transcribable polynucleotide molecules and have a substantial percent sequence identity to the polynucleotide sequences of the polynucleotide molecules are described herein."Homology" refers to the level of similarity between two or more nucleic acid or amino acid sequences in terms of percent of positional identity (i.e., sequence similarity or identity).

Homology also refers to the concept of similar functional properties among different nucleic acids or proteins.

Any number of methods well known to those skilled in the art can be used to isolate fragments of a polynucleotide molecule disclosed herein. For example, PCR (polymerase chain reaction) technology can be used to amplify flanking regions from a genomic library of a plant using publicly available sequence information. A number of methods are known to those of skill in the art to amplify unknown DNA sequences adjacent to a core region of known sequence. Methods include inverse PCR (IPCR), vectorette PCR, Y-shaped PCR, and genome walking approaches. Polynucleotide molecule fragments can also be obtained by other techniques such as by directly synthesizing the fragment by chemical means, as is commonly practiced by using an automated oligonucleotide synthesizer. For the present invention, the polynucleotide molecules were isolated by designing PCR primers based an available sequence information.

Those of skill in the art are familiar with the standard resource materials that describe specific conditions and procedures for the construction, manipulation, and isolation of macromolecules (e.g., polynucleotide molecules, plasmids), as well as the generation of recombinant organisms and the screening and isolation of polynucleotide molecules.

### Polynucleotide Constructs

Any of the ANT1-NCRE molecules and transcribable polynucleotide molecule sequences described above may be provided in a construct. Constructs would typically contain a promoter, such as provided in SEQ ID NO: 1 or SEQ ID NO: 2, operably linked to a transcribable polynucleotide molecule.

As used herein, the term "construct" or refers to any recombinant polynucleotide molecule such as a plasmid, cosmid, virus, autonomously replicating polynucleotide molecule, phage, or linear or circular single-stranded or double-stranded DNA or RNA polynucleotide molecule, derived from any source, capable of genomic integration or autonomous replication, comprising a polynucleotide molecule where one or more polynucleotide molecule has been linked in a functionally operative manner, i.e. operably linked.

As used herein, the term "vector" or "vector construct" refers to any recombinant polynucleotide construct that may be used for the purpose of transformation, i.e. the introduction of heterologous DNA into a host cell.

In addition, constructs may include additional polynucleotide molecules from the 3'-untranslated region (3' UTR) of plant genes, *e.g.* a 3' UTR, such as the PI-II termination region of potato, pea rubisco small subunit 3'UTR or the octopine or nopaline synthase 3'termination regions. In addition, constructs may include additional polynucleotide molecules from the 5'-untranslated region (5' UTR) of plant genes which can play an important role in translation initiation and can also be a genetic component in a plant expression construct, such as provided in SEQ ID NO: 1 or SEQ ID NO: 2, *e.g.* a leader to enhance transgene expression, such as non-translated 5' leader polynucleotide molecules derived from heat shock protein genes which have been demonstrated to enhance gene expression in plants (see for example, U.S. Patent Nos. 5,659,122 and 5,362,865). In addition, constructs may include additional polynucleotide molecules such as introns, *e.g.,* the first intron of the actin 1 gene from *Oryza sativa (*U.S. Patent No. 5,641,876), the IS50L intron from the light sensitive 1 gene of *Solanum tuberosum,* the intron of the heat shock protein 70 gene of *Petunia hybrida* (U.S. Patent No. 5,659,122), the Hsp70 intron of the Heat shock protein 70 gene of *Zea mays* (U.S. Patent No. 5,593,874). These additional polynucleotide molecules may be derived from a source that is native or heterologous with respect to the other elements present in the construct.

Means for preparing recombinant constructs are well known in the art. Methods for making recombinant vector constructs particularly suited to plant transformation include, without limitation, those described in U.S. Patent Nos. 4,971,908, 4,940,835, 4,769,061 and 4,757,011. These type of vectors have also been reviewed (Rodriguez, et al. Vectors: A Survey of Molecular Cloning Vectors and Their Uses, Butterworths, Boston, 1988; Glick et al., Methods in Plant Molecular Biology and Biotechnology, CRC Press, Boca Raton, Fla., 1993).

Typical constructs useful for expression of nucleic acids in higher plants are well known in the art and include vectors derived from the tumor-inducing (Ti) plasmid of *Agrobacterium tumefaciens* (Rogers, et al., Meth. In Enzymol, 153: 253-277, 1987). Other recombinant constructs useful for plant transformation, including the pCaMVCN transfer control vector, have also been described (Fromm et al., Proc. Natl. Acad. Sci. USA, 82(17): 5824-5828, 1985).

### Promoters in the Recombinant Constructs

The promoter used in the recombinant construct preferably transcribes a heterologous transcribable polynucleotide molecule sequence at a high level in a plant. More preferably, the promoter comprises a nucleic acid sequence that exhibits 95% or greater, 96 or greater, 97 or greater, 98 or greater, or 99% or greater identity to the full-length nucleic acid sequence of SEQ ID NO:1, or any complements thereof, or any fragments thereof. The promoter most preferably comprises a nucleic acid sequence selected from SEQ ID NO:1 or SEQ ID NO:2.

### Additional Promoters in the Recombinant Construct

One or more additional promoters may also be provided in the recombinant construct. These promoters may be operably linked to any of the transcribable polynucleotide molecule sequences described above. Alternatively, the promoters may be operably linked to other nucleic acid sequences, such as those encoding transit peptides, selectable marker proteins, or antisense sequences.

As used herein, the term "chimeric" refers to the product of the fusion of portions of two or more different polynucleotide molecules. As used herein, the term "chimeric promoter" refers to a promoter produced through the manipulation of known promoters or other polynucleotide molecules. Such chimeric promoters may combine enhancer domains that can confer or modulate gene expression from one or more promoters or regulatory elements, for example, by fusing a heterologous enhancer domain from a first promoter to a second promoter with its own partial or complete regulatory elements. Thus, the design, construction, and use of chimeric promoters according to the methods disclosed herein for modulating the expression of operably linked polynucleotide sequences are encompassed by the present invention.

Novel chimeric promoters can be designed or engineered by a number of methods. For example, a chimeric promoter may be produced by fusing an enhancer domain from a first promoter to a second promoter. The resultant chimeric promoter may have novel expression properties relative to the first or second promoters. Novel chimeric promoters can be constructed such that the enhancer domain from a first promoter is fused at the 5' end, at the 3' end, or at any position internal to the second promoter. For example, one or more caulimovirus enhancer elements fused to the promoter of the present invention. The location of the enhancer domain fusion relative to the second promoter may cause the resultant chimeric promoter to have novel expression properties relative to a fusion made at a different location. Methods for making chimeric promoters particularly suited to plant transformation include, those described in U.S. Patent No. 6,660,911.

These additional promoters may be selected on the basis of the cell type into which the vector construct will be inserted. Promoters which function in bacteria, yeast, and plants are all well taught in the art. The additional promoters may also be selected on the basis of their regulatory features. Examples of such features include enhancement of transcriptional activity, inducibility, tissue-specificity, and developmental stage-specificity. In plants, promoters that are inducible, of viral or synthetic origin, constitutively active, temporally regulated, and spatially regulated have been described (Poszkowski, et al., EMBO J., 3: 2719, 1989; Odell, et al., Nature, 313:810, 1985; Chau et al., Science, 244:174-181. 1989).

Often-used constitutive promoters include the CaMV 35S promoter, the enhanced CaMV 35S promoter, the Figwort Mosaic Virus (FMV) promoter, the mannopine synthase *(mas)* promoter, the nopaline synthase *(nos)* promoter, and the octopine synthase (ocs) promoter. Useful inducible promoters include promoters induced by salicylic acid or polyacrylic acids induced by application of safeners (substituted benzenesulfonamide herbicides), heat-shock promoters, a nitrate-inducible promoter derived from the spinach nitrite reductase transcribable polynucleotide molecule sequence, hormone-inducible promoters, and light-inducible promoters associated with the small subunit of RuBP carboxylase and LHCP families.

Examples of useful tissue-specific, developmentally-regulated promoters include the ß-conglycinin 7Sα, promoter, and seed-specific promoters. Plant functional promoters useful for preferential expression in seed plastid include those from plant storage proteins and from proteins involved in fatty acid biosynthesis in oilseeds. Examples of such promoters include the 5' regulatory regions from such transcribable polynucleotide molecule sequences as napin, phaseolin, zein, soybean trypsin inhibitor, ACP, stearoyl-ACP desaturase, and oleosin. Seed-specific regulation is discussed in EP 0 255 378. Another exemplary tissue-specific promoter is the lectin promoter, which is specific for seed tissue.

Particularly preferred additional promoters in the recombinant construct include the nopaline synthase *(nos),* mannopine synthase *(mas),* and octopine synthase *(ocs)* promoters, which are carried an tumor-inducing plasmids of *Agrobacterium tumefaciens;* the cauliflower mosaic virus (CaMV) 19S and 35S promoters; the enhanced CaMV 35S promoter; the Figwort Mosaic Virus (FMV) 35S promoter; the light-inducible promoter from the small subunit of ribulose-1,5-bisphosphate carboxylase (ssRUBISCO); the EIF-4A promoter from tobacco (Mandel, et al., Plant Mol. Biol, 29: 995-1004, 1995); corn sucrose synthetase ; corn alcohol dehydrogenase 1; corn light harvesting complex; corn heat shock protein; the chitinase promoter from *Arabidopsis;* the LTP (Lipid Transfer Protein) promoters from broccoli; petunia chalcone isomerase; bean glycine rich protein 1; Potato patatin; the ubiquitin promoter from maize; and the actin promoter from corn.

The additional promoter is preferably seed selective, tissue selective, constitutive, or inducible. The promoter is most preferably the nopaline synthase (NOS), octopine synthase (OCS), mannopine synthase (MAS), cauliflower mosaic virus 19S and 35S (CaMV19S, CaMV35S), enhanced CaMV (eCaMV), ribulose 1,5-bisphosphate carboxylase (ssRUBISCO), figwort mosaic virus (FMV), CaMV derived AS4, tobacco RB7, wheat POX1, tobacco EIF-4, lectin protein (Le 1), or corn RC2 promoter.

### Other Elements in the Recombinant Construct

The composition and utility for constructs comprising the Adenylate Translocator protein gene non-coding regulatory element molecules, hereinafter referred to as ANT1-NCRE molecules are described herein. These constructs may include 5' non-translated leader sequences.

Various cis-acting untranslated 5' and 3' non-coding regulatory element sequences may be included in the recombinant nucleic acid construct. Any such regulatory sequences may be provided in a recombinant construct with other regulatory sequences. Such combinations can be designed or modified to produce desirable regulatory features.

5' non-coding regulatory element sequences typically comprise non-translated leader sequences and/or introns.

A 3' non-translated region typically provides a transcriptional termination signal, and a polyadenylation signal which functions in plants to cause the addition of adenylate nucleotides to the 3' end of the mRNA. These may be obtained from the 3' regions to the nopaline synthase *(nos)* coding sequence, the soybean 7Sα storage protein coding sequence, the albumin coding sequence, and the pea ssRUBISCO E9 coding sequence. Particularly preferred 3' nucleic acid sequences include *nos* 3', E9 3', ADR12 3', 7Sα 3', 11S 3', and albumin 3'.

Typically, nucleic acid sequences located a few hundred base pairs downstream of the polyadenylation site serve to terminate transcription. These regions are required for efficient polyadenylation of transcribed mRNA.

The recombinant construct may further comprise a nucleic acid sequence encoding a transit peptide. This peptide may be useful for directing a protein to the extracellular space, a chloroplast, or to some other compartment inside or outside of the cell (see, e.g., EP-A-0218571).

### Transcribable Polynucleotide Molecule Sequences in the Recombinant Nucleic Acid Construct

The non-coding regulatory element (NCRE) in the recombinant construct is preferably operably linked to a transcribable polynucleotide molecule sequence. Exemplary transcribable polynucleotide molecule sequences, and modified forms thereof, are described in detail above. The NCRE molecules of the present invention may be operably linked to a transcribable polynucleotide molecule sequence that is heterologous with respect to the NCRE. In one aspect, the transcribable polynucleotide molecule sequence may generally be any nucleic acid sequence for which an increased level of transcription is desired. The transcribable polynucleotide molecule sequence preferably encodes a polypeptide that is suitable for incorporation into the diet of a human or an animal. Suitable transcribable polynucleotide molecule sequences include those encoding a yield protein, a stress resistance protein, a developmental control protein, a tissue differentiation protein, a meristem protein, an environmentally responsive protein, a senescence protein, a hormone responsive protein, an abscission protein, a source protein, a sink protein, a flower control protein, a seed protein, an herbicide resistance protein, a disease resistance protein, a fatty acid biosynthetic enzyme, a tocopherol biosynthetic enzyme, an amino acid biosynthetic enzyme, and an insecticidal protein.

Alternatively, the NCRE or the transcribable polynucleotide molecule sequences may be designed to down-regulate a specific nucleic acid sequence. This is typically accomplished by linking the NCRE to a transcribable polynucleotide molecule sequence that is oriented in the antisense direction. One of ordinary skill in the art is familiar with such antisense technology. Using such an approach, a cellular nucleic acid sequence is effectively down regulated as the subsequent steps of translation are disrupted. Nucleic acid sequences may be negatively regulated in this manner. Thus a polynucleotide molecule such as provided in SEQ ID NO:1 or SEQ ID NO: 2 that is operably linked to a transcribable polynucleotide molecule so as to direct transcription of the transcribable polynucleotide molecule at a desired level or in a desired tissue or developmental pattern upon introduction of the construct into a plant cell is described. In some cases, the transcribable polynucleotide molecule comprises a protein-coding region of a gene, and a non-coding regulatory element of SEQ ID NO:1 or SEQ ID NO: 2 provides for transcription of a functional mRNA molecule that is translated and expressed as a protein product. Constructs may also be constructed for transcription of antisense RNA molecules or other similar inhibitory RNA molecules in order to suppress expression of a specific gene of interest in a target host cell.

Exemplary transcribable polynucleotide molecules for incorporation into constructs of the present invention include, for example, DNA molecules or genes from a species other than the target gene species, or even genes that originate with or are present in the same species, but are incorporated into recipient cells by genetic engineering methods rather than classical reproduction or breeding techniques. Exogenous gene or genetic element is intended to refer to any gene or polynucleotide molecule that is introduced into a recipient cell. The type of polynucleotide molecule included in the exogenous DNA can include DNA that is already present in the plant cell, DNA from another plant, DNA from a different organism, or a DNA generated externally, such as a DNA molecule containing an antisense message of a gene, or a DNA molecule encoding an artificial or modified version of a gene.

### Fusion Proteins

Any of the above described structural nucleic acid sequences, and modified forms thereof, may be linked with additional nucleic acid sequences to encode fusion proteins. The additional nucleic acid sequence preferably encodes at least 1 amino acid, peptide, or protein.

Production of fusion proteins is routine in the art and many possible fusion combinations exist.

For instance, the fusion protein may provide a "tagged" epitope to facilitate detection of the fusion protein, such as GST, GFP, FLAG, or polyHIS. Such fusions preferably encode between 1 and 50 amino acids, more preferably between 5 and 30 additional amino acids, and even more preferably between 5 and 20 amino acids.

Alternatively, the fusion may provide regulatory, enzymatic, cell signaling, or intercellular transport functions. For example, a sequence encoding a chloroplast transit peptide may be added to direct a fusion protein to the chloroplasts within a plant cell. Such fusion partners preferably encode between 1 and 1000 additional amino acids, more preferably between 5 and 500 additional amino acids, and even more preferably between 10 and 250 amino acids.

Where plastid targeting is necessary, for example, the EPSPS enzyme functions in a plant chloroplast, therefore, DNA molecules encoding a chloroplast transit peptide (CTP) are engineered into a DNA molecule encoding an EPSPS protein to encode a fusion protein of the CTP to the N terminus of an EPSPS creating a chimeric molecule. A chimeric polynucleic acid coding sequence is comprised of two or more open reading frames joined in-frame that encode a chimeric protein, for example, a chloroplast transit peptide and an EPSPS enzyme. A chimeric gene refers to the multiple genetic elements derived from heterologous sources operably linked to comprise a gene. In the present invention the DNA construct expresses a chimeric CTP-EPSPS protein that directs the glyphosate resistant EPSPS protein into the plant chloroplast. In a native plant EPSPS gene, chloroplast transit peptide regions are contained in the native coding sequence (for example, CTP2, Klee et al., Mol. Gen. Genet. 210:47-442,1987). The CTP is cleaved from the EPSPS enzyme at the chloroplast membrane to create a "mature EPSPS or EPSPS enzyme" that refers to the polypeptide sequence of the processed protein product remaining after the chloroplast transit peptide has been removed. The production of glyphosate tolerant plants by expression of a fusion protein comprising an amino-terminal CTP with a glyphosate resistant EPSPS enzyme is well known by those skilled in the art, (U.S. Patent Nos. 5,627,061, 5,633,435 and 5,312,910, EP 0218571, EP 189707, EP 508909, and EP 924299). Those skilled in the art will recognize that various chimeric constructs can be made that utilize the functionality of a particular CTP to import glyphosate resistant EPSPS enzymes into the plant cell chloroplast. The present invention illustrates the utility of the combination of the Ant1 promoter molecule operably linked to a DNA molecule that encodes for a chloroplast transit peptide fused to an EPSP synthase.

### NCRE molecules and Genes of Agronomie Interest

The recombinant construct may also contain one or more additional transcribable polynucleotide molecule sequences. These additional transcribable polynucleotide molecule sequences may generally be any sequences suitable for use in a recombinant construct. Such transcribable polynucleotide molecule sequences include any of the transcribable polynucleotide molecule sequences , and modified forms thereof, described above. The additional transcribable polynucleotide molecule sequences may also be operably linked to any of the above described NCRE sequences. The one or more transcribable polynucleotide molecule sequences may each be operably linked to separate NCRE sequences: Alternatively, the transcribable polynucleotide molecule sequences may be operably linked to a single NCRE *(i.e.* a single operon).

As used herein, the term "gene of agronomic interest" refers to a transcribable polynucleotide molecule that includes a gene that provides a desirable characteristic associated with plant morphology, physiology, growth and development, yield, nutritional enhancement, disease or pest resistance, or environmental or chemical tolerance.

The additional transcribable polynucleotide molecule sequences preferably encode a gene of agronomic interest, such as : a yield protein, a stress resistance protein, a developmental control protein, a tissue differentiation protein, a meristem protein, an environmentally responsive protein, a senescence protein, a hormone responsive protein, an abscission protein, a source protein, a sink protein, a flower control protein, a seed protein, an herbicide resistance protein, a disease resistance protein, a fatty acid biosynthetic enzyme, a tocopherol biosynthetic enzyme, an amino acid biosynthetic enzyme, and an insecticidal protein. Alternatively, the additional transcribable polynucleotide molecule sequences may be designed to down-regulate a specific nucleic acid sequence. This is typically accomplished by operably linking the second structural amino acid, in an antisense orientation, with a promoter.

One of ordinary skill in the art is familiar with such antisense technology. The process is also briefly described above. Any nucleic acid sequence may be negatively regulated in this manner.

In one aspect, a polynucleotide molecule as shown in SEQ ID NO:1 or SEQ ID NO: 2, comprising regulatory elements, such as a promoter, leader, or an intron element, is incorporated into a construct such that a polynucleotide molecule is operably linked to a transcribable polynucleotide molecule that is a gene of agronomic interest.

The expression of a gene of agronomic interest is desirable in order to confer an agronomically important trait. A gene of agronomic interest that provides a beneficial agronomic trait to crop plants may be, for example, including genetic elements comprising herbicide resistance (U.S. Patent Nos. 5,633,435 and 5,463,175), increased yield (U.S. Patent No. 5,716,837), insect control (U.S. Patent Nos. 6,063,597; 6,063,756; 6,093,695; 5,942,664; and 6,110,464), fungal disease resistance (U.S. Patent Nos. 5,516,671; 5,773,696; 6,121,436; 6,316,407, and 6,506,962), virus resistance (U.S. Patent Nos. 5,304,730 and 6,013,864), nematode resistance (U.S. Patent No. 6,228,992), bacterial disease resistance (U.S. Patent No. 5,516,671), starch production (U.S. Patent Nos. 5,750,876 and 6,476,295), modified oils production (U.S. Patent No. 6,444,876), high oil production (U.S. Patent Nos. 5,608,149 and 6,476,295), modified fatty acid content (U.S. Patent No. 6,537,750), high protein production (U.S. Patent No. 6,380,466), fruit ripening (U.S. Patent No. 5,512,466), enhanced animal and human nutrition (U.S. Patent Nos. 5,985,605 and 6,171,640), biopolymers (U.S. Patent No. 5,958,745 and U.S. Patent Publication No. US20030028917), environmental stress resistance (U.S. Patent No. 6,072,103), pharmaceutical peptides (U.S. Patent No. 6,080,560), improved processing traits (U.S. Patent No. 6,476,295), improved digestibility (U.S. Patent No. 6,531,648) low raffinose (U.S. Patent No. 6,166,292), industrial enzyme production (U.S. Patent No. 5,543,576), improved flavor (U.S. Patent No. 6,011,199), nitrogen fixation (U.S. Patent No. 5,229,114), hybrid seed production (U.S. Patent No. 5,689,041), and biofuel production (U.S. Patent No. 5,998,700).

Alternatively, a transcribable polynucleotide molecule can effect the above mentioned phenotypes by encoding a RNA molecule that causes the targeted suppression of expression of an endogenous gene, for example via antisense, dsRNA, or cosuppression-mediated mechanisms. The RNA could also be a catalytic RNA molecule (i.e., a ribozyme) engineered to cleave a desired endogenous mRNA product. Thus, any polynucleotide molecule that encodes a protein or mRNA that expresses a phenotype or morphology change of interest may be useful for the practice of the present invention.

The constructs are generally double Ti plasmid border DNA constructs that have the right border (RB or AGRtu.RB) and left border (LB or AGRtu.LB) regions of the Ti plasmid isolated from *Agrobacterium tumefaciens* comprising a T-DNA, that along with transfer molecules provided by the *Agrobacterium* cells, permits the integration of the T-DNA into the genome of a plant cell. The constructs also contain the plasmid backbone DNA segments that provide replication function and antibiotic selection in bacterial cells, for example, an *E. coli* origin of replication such as *ori*322, a broad host range origin of replication such as *oriV, rop,* or *oriRi,* and a coding region for a selectable marker such as Spec/Strp that encodes for Tn7 aminoglycoside adenyltransferase *(aadA)* conferring resistance to spectinomycin or streptomycin, or a gentamicin (Gm, Gent) selectable marker gene. For plant transformation, the host bacterial strain is often *Agrobacterium tumefaciens* ABI, C58, or LBA4404, however, other strains known to those skilled in the art of plant transformation can function in the present invention.

### Selectable Markers

The polynucleotide molecules of the present invention can be incorporated into a DNA construct using marker genes as described and tested in transient analyses that provide an indication of gene expression in stable plant systems.

As used herein the term "marker gene" or "selectable marker" refers to any transcribable polynucleotide molecule whose expression can be screened for or scored in some way. Included within the term "selectable markers" is also genes which encode a secretable marker whose secretion can be detected as a means of identifying or selecting for transformed cells.

Methods of testing for marker gene expression in transient assays are known to those of skill in the art. Transient expression of marker genes has been reported using a variety of plants, tissues, and DNA delivery systems. For example, types of transient analyses can include direct gene delivery via electroporation or particle bombardment of tissues in any transient plant assay using any plant species of interest. Such transient systems would include electroporation of protoplasts from a variety of tissue sources or particle bombardment of specific tissues of interest. The present invention encompasses the use of any transient expression system to evaluate polynucleotide molecule operably linked to any transcribable polynucleotide molecules, including marker genes, or genes of agronomic interest. Examples of plant tissues envisioned to test in transients via an appropriate delivery system would include leaf base tissues, callus, cotyledons, roots, endosperm, embryos, floral tissue, pollen, and epidermal tissue.

The recombinant construct may further comprise a selectable marker. The nucleic acid sequence serving as the selectable marker functions to produce a phenotype in cells which facilitates their identification relative to cells not containing the marker.

Examples of selectable markers include a neo gene, which codes for kanamycin resistance and can be selected for using kanamycin, G418; a bar gene which codes for bialaphos resistance; a mutant EPSP synthase gene which encodes glyphosate resistance; a nitrilase gene which confers resistance to bromoxynil; a mutant acetolactate synthase gene (ALS) which confers imidazolinone or sulphonylurea resistance (EP-A-0154204); green fluorescent protein (GFP); and a methotrexate resistant DHFR gene.

Other exemplary selectable markers include: a ß-glucuronidase or uidA gene (GUS), which encodes an enzyme for which various chromogenic substrates are known; an R-locus gene, which encodes a product that regulates the production of anthocyanin pigments in plant tissues; a ß-lactamase gene, which encodes an enzyme for which various chromogenic substrates are known *(e.g*., PADAC, a chromogenic cephalosporin); a luciferase gene; a xylE gene which encodes a catechol dioxygenase that can convert chromogenic catechols; an α-amylase gene; a tyrosinase gene, which encodes an enzyme capable of oxidizing tyrosine to DOPA and dopaquinone (which in turn condenses to melanin); and an α-galactosidase, which will turn a chromogenic α-galactose substrate.

Included within the term "selectable markers" are also genes which encode a secretable marker whose secretion can be detected as a means of identifying or selecting for transformed cells. Examples include markers that encode a secretable antigen that can be identified by antibody interaction, or even secretable enzymes which can be detected catalytically. Selectable secreted marker proteins fall into a number of classes, including small, diffusible proteins which are detectable, *(e.g.,* by ELISA), small active enzymes which are detectable in extracellular solution *(e.g.,* α-amylase, ß-lactamase, phosphinothricin transferase), or proteins which are inserted or trapped in the cell wall (such as proteins which include a leader sequences uch as that found in the expression unit of extension or tobacco PR-S). Other possible selectable marker genes will be apparent to those of skill in the art.

The selectable marker is preferably GUS, green fluorescent protein (GFP), neomycin phosphotransferase II *(nptII),* luciferase (LUX), an antibiotic resistance coding sequence, or an herbicide *(e.g.,* glyphosate) resistance coding sequence. The selectable marker is most preferably a kanamycin, hygromycin, or herbicide resistance marker.

Any scorable or screenable marker gene can be used in a transient assay. Exemplary marker genes for transient analyses of the promoters or promoter fragments of the present invention include a GUS gene (U.S. Patent No. 5,599,670) or a GFP gene (U.S. Patent No. 5,491,084). The constructs containing the polynucleotide molecule operably linked to a marker gene are delivered to the tissues and the tissues are analyzed by the appropriate mechanism, depending an the marker. The quantitative or qualitative analyses are used as a tool to evaluate the potential expression profile of the polynucleotide molecule when operably linked to genes of agronomic interest in stable plants.

Thus, in one aspect a polynucleotide molecule, such as shown in SEQ ID NO:1 or SEQ ID NO: 2, is incorporated into a DNA construct such that a polynucleotide molecule of the present invention is operably linked to a transcribable polynucleotide molecule that provides for a selectable, screenable, or scorable marker. Markers for use in the practice of the present invention include transcribable polynucleotide molecules encoding ß-glucuronidase (GUS), green fluorescent protein (GFP), luciferase (LUC), proteins that confer antibiotic resistance, or proteins that confer herbicide tolerance. Useful antibiotic resistance markers, including those encoding proteins conferring resistance to kanamycin *(nptII),* hygromycin B *(aph IV),* streptomycin or spectinomycin *(aad,* spec/strep) and gentamycin *(aac3* and *aacC4)* are known in the art.

Herbicides for which transgenic plant tolerance has been demonstrated and the method of the present invention can be applied, include : glyphosate, glufosinate, sulfonylureas, imidazolinones, bromoxynil, delapon, cyclohezanedione, protoporphyrionogen oxidase inhibitors, and isoxasflutole herbicides.

"Glyphosate" refers to N-phosphonomethylglycine and its salts, Glyphosate is the active ingredient of Roundup^{®} herbicide (Monsanto Co.). Plant treatments with "glyphosate" refer to treatments with the Roundup^{®} or Roundup Ultra^{®} herbicide formulation, unless otherwise stated. Glyphosate as N-phosphonomethylglycine and its salts (not formulated Roundup® herbicide) are components of synthetic culture media used for the selection of bacteria and plant tolerance to glyphosate or used to determine enzyme resistance in *in vitro* biochemical assays.

Examples of commercial formulations of glyphosate include those sold by Monsanto Company as ROUNDUP^{®}, ROUNDUP^{®} ULTRA, ROUNDUP^{®} ULTRAMAX, ROUNDUP^{®}, ROUNDUP^{®} CT, ROUNDUP^{®} EXTRA, ROUNDUP^{®} BIACTIVE, ROUNDUP^{®} BIOFORCE, RODEO^{®},

POLARIS^{®}, SPARK^{®} and ACCORD^{®} herbicides, all of which contain glyphosate as its isopropylammonium salt; WEATHERMAX^{®}, which contains glyphosate as its potassium salt; those sold by Monsanto Company as ROUNDUP^{®} DRY and RIVAL^{®} herbicides, which contain glyphosate as its ammonium salt; that sold by Monsanto Company as ROUNDUP^{®} GEOFORCE, which contains glyphosate as its sodium salt; and that sold by Zeneca Limited as TOUCHDOWN^{®} herbicide, which contains glyphosate as its trimethylsulfonium salt.

Polynucleotide molecules encoding proteins involved in herbicide tolerance are known in the art, and include a polynucleotide molecule encoding 5-enolpyruvylshikimate-3-phosphate synthase (EPSPS) described in U.S. Patent Nos. 5,627,061, 5,633,435, 6,040,497 and 5,094,945 for glyphosate tolerance; polynucleotides encoding a glyphosate oxidoreductase and a glyphosate-N-acetyl transferase (GOX, U.S. Patent 5,463,175 and GAT, U.S. Patent publication 20030083480); a polynucleotide molecule encoding bromoxynil nitrilase *(Bxn)* described in U.S. Patent No. 4,810,648 for Bromoxynil tolerance; a polynucleotide molecule encoding phytoene desaturase *(crtI)* described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:2188-2193 for tolerance to sulfonylurea herbicides; and the *bar* gene described in DeBlock, et al. (1987) EMBO J. 6:2513-2519 for glufosinate and bialaphos tolerance; resistant hydroxyphenyl pyruvate dehydrogenase (HPPD, U.S. Patent 6,768,044). The promoter of the present invention can express genes that encode for phosphinothricin
acetyltransferase, glyphosate resistant EPSPS, aminoglycoside phosphotransferase, hydroxyphenyl pyruvate dehydrogenase, hygromycin phosphotransferase, neomycin phosphotransferase, dalapon dehalogenase, bromoxynil resistant nitrilase, anthranilate synthase, glyphosate oxidoreductase and glyphosate-N-acetyl transferase.

### Transformed Plants And Plant Cells

A method of producing transformed cells which comprise, in a 5' to 3' orientation, one or more non-coding regulatory elements operably linked to a heterologous transcribable polynucleotide molecule sequence is described herein . Other sequences may also be introduced into the
cell along with the non-coding regulatory elements and transcribable polynucleotide molecule sequence. These other sequences may include 3' transcriptional terminators, 3' polyadenylation signals, other untranslated sequences, transit or targeting sequences, selectable markers, enhancers, and operators.

The method of transformation generally comprises the steps of selecting a suitable host cell, transforming the host cell with a recombinant vector, and obtaining the transformed host cell. As used herein, the term "transformed" refers to a cell, tissue, organ, or organism into which has been introduced a foreign polynucleotide molecule, such as a construct. The introduced polynucleotide molecule may be integrated into the genomic DNA of the recipient cell, tissue, organ, or organism such that the introduced polynucleotide molecule is inherited by subsequent progeny. A "transgenic" or "transformed" cell or organism also includes progeny of the cell or organism and progeny produced from a breeding program employing such a transgenic plant as a parent in a cross and exhibiting an altered phenotype resulting from the presence of a foreign polynucleotide molecule.

Preferred recombinant constructs, transcribable polynucleotide molecule sequences, non-coding regulatory elements, and other regulatory elements are described above. The non-coding regulatory element preferably has a nucleic acid sequence that exhibits 95% or greater, 96 or greater, 97 or greater, 98 or greater, or 99% or greater identity to the full-length sequence of SEQ ID NO:1. The recombinant construct used to transform the host cell typically comprises, in a 5' to 3' orientation: a promoter to direct the transcription of a transcribable polynucleotide molecule sequence , a transcribable polynucleotide molecule sequence , a 3' transcriptional terminator, and a 3' polyadenylation signal. The recombinant vector may further comprise untranslated nucleic acid sequences, transit and targeting nucleic acid sequences, selectable markers, enhancers, or operators. Suitable recombinant vectors, transcribable polynucleotide molecule sequences , promoters, and other regulatory elements include those described above.

Technology for introduction of DNA into cells is well known to those of skill in the art. These methods can generally be classified into five categories: (1) chemical methods (Graham and Van der Eb, Virology, 54(2): 536-539, 1973; Zatloukal, et al., Ann. N.Y. Acad. Sci., 660: 136-153, 1992); (2) physical methods such as microinjection (Capecchi, Cell, 22(2): 479-488, 1980), electroporation (Wong and Neumann, Biochim. Biophys. Res. Commun., 107(2): 584-587, 1982; Fromm et al., Proc. Natl. Acad. Sci. USA, 82(17): 5824-5828, 1985; U.S. Patent No. 5,384,253) and particle acceleration (Johnston and Tang, Methods Cell Biol., 43(A): 353-365, 1994; Fynan et al., Proc. Natl. Acad. Sci. USA, 90(24): 11478-11482, 1993); (3) viral vectors (Clapp, Clin. Perinatol., 20(1): 155-168, 1993; Lu, et al., J. Exp. Med., 178(6): 2089-2096, 1993; Eglitis and Anderson, Biotechniques, 6(7): 608-614, 1988); (4) receptor-mediated mechanisms (Curiel et al., Hum. Gen. Ther., 3(2):147-154, 1992; Wagner, et al., Proc. Natl. Acad. Sci. USA, 89(13): 6099-6103, 1992), and (5) bacterial mediated mechanisms such as with Agrobacterium. Alternatively, nucleic acids can be directly introduced into pollen by directly injecting a plant's reproductive organs (Zhou, et al., Methods in Enzymology, 101: 433, 1983; Hess, Intern Rev. Cytol., 107: 367, 1987; Luo, et al., Plant Mol Biol. Reporter, 6: 165, 1988; Pena, et al., Nature, 325: 274, 1987). Other transformation methods include, for example, protoplast transformation as illustrated in U.S. Patent No. 5,508,184. The nucleic acids may also be injected into immature embryos (Neuhaus, et al., Theor. Appl. Genet., 75: 30, 1987).

The most commonly used methods for transformation of plant cells are the Agrobacterium-mediated DNA transfer process (Fraley et al., Proc. Natl. Acad. Sci. U.S.A., 80: 4803, 1983) (as illustrated in U.S. Patent Nos. 5,824,877; 5,591,616; 5,981,840; and 6,384,301) and the biolistics or microprojectile bombardment mediated process (i.e. the *gene gun)* (such as described in U.S. Patent Nos. 5,015,580; 5,550,318; 5,538,880; 6,160,208; 6,399,861; and 6,403,865). Typically, nuclear transformation is desired but where it is desirable to specifically transform plastids, such as chloroplasts or amyloplasts, plant plastids may be transformed utilizing a microprojectile mediated delivery of the desired polynucleotide for certain plant species such as tobacco, Arabidopsis, potato and Brassica species.

Agrobacterium-mediated transformation is achieved through the use of a genetically engineered soil bacterium belonging to the genus Agrobacterium. Several Agrobacterium species mediate the transfer of a specific DNA known as "T-DNA", that can be genetically engineered to carry any desired piece of DNA into many plant species. The major events marking the process of T-DNA mediated pathogenesis are: induction of virulence genes, processing and transfer of T-DNA. This process is the subject of many reviews (Ream, Ann. Rev. Phytopathol. 27: 583-618, 1989; Howard and Citovsky, Bioassays, 12:103-108, 1990; Kado, Crit. Rev. Plant Sci. 10:1-32, 1991; Zambryski, Annual Rev. Plant Physiol. Plant Mol. Biol., 43: 465-490, 1992; Gelvin, In Transgenic Plants, Kung and Wu eds., Academic Press, San Diego, pp. 49-87, 1993; Binns and Howitz, 1994, In Bacterial Pathogenesis of Plants and Animals (Dang, ed.). Berlin: Springer Verlag, pp. 119-138, 1994; Hooykaas and Beijersbergen, Ann. Rev. Phytopathol. 32:157-179, 1994; Lessl and Lanka, Cell 77:321-324, 1994; Zupan and Zambryski, Annual Rev. Phytopathol. 27, 583-618, 1995).

With respect to microprojectile bombardment (U.S. Patent Nos. 5,550,318; 5,538,880; 5,610,042; and WO 95/06128), particles are coated with nucleic acids and delivered into cells by a propelling force. Exemplary particles include those comprised of tungsten, platinum, and preferably, gold. For the bombardment, cells in suspension are concentrated on filters or solid culture medium. Alternatively, immature embryos or other target cells may be arranged on solid culture medium. The cells to be bombarded are positioned at an appropriate distance below the microprojectile stopping plate.

Microprojectile bombardment techniques are widely applicable, and may be used to transform virtually any plant species. Examples of species that have been transformed by microprojectile bombardment include monocot species such as maize (WO 95/06128), barley (Ritala et al., 1994; Hensgens et al., 1993), wheat (U.S. Patent No. 5,563,055), rice (Hensgens et al., 1993), oat (Torbet et al., 1995; Torbet et al., 1998), rye (Hensgens et al., 1993), sugarcane (Bower et al., 1992), and sorghum (Casa et al., 1993; Hagio et al., 1991); as well as a number of dicots including tobacco (Tomes et al., 1990; Buising and Benbow, 1994), soybean (U.S. Patent No. 5,322,783), sunflower (Knittel et al., 1994), peanut (Singsit et al., 1997), cotton (McCabe and Martinell, 1993), tomato (Van Eck et al., 1995), and legumes in general (U.S. Patent No. 5,563,055). To select or score for transformed plant cells regardless of transformation methodology, the DNA introduced into the cell contains a gene that functions in a regenerable plant tissue to produce a compound that confers upon the plant tissue resistance to an otherwise toxic compound. Genes of interest for use as a selectable, screenable, or scorable marker will include GUS, green fluorescent protein (GFP), luciferase (LUX), antibiotic or herbicide tolerance genes. Examples of antibiotic resistance genes include the penicillins, kanamycin (and neomycin, G418, bleomycin); methotrexate (and trimethoprim); chloramphenicol; kanamycin and tetracycline.

Particularly preferred selectable marker genes for use in the present invention will include genes that confer resistance to compounds such as antibiotics like kanamycin (nptII), hygromycin B (aph IV) and gentamycin (aac3 and aacC4) (Dekeyser et al., Plant Physiol., 90:217-223, 1989), and herbicides like glyphosate (Della-Cioppa et al., Bio/Technology, 5:579-584, 1987). Other selection devices can also be implemented including tolerance to phosphinothricin, bialaphos, and positive selection mechanisms (Joersbo et al., Mol. Breed., 4:111-117, 1998).

The transformed cells, identified by selection or screening and cultured in an appropriate medium that supports regeneration, will then be allowed to mature into plants.

The present invention can be used with any transformable cell or tissue. By transformable as used herein is meant a cell or tissue that is capable of further propagation to give rise to a plant. Those of skill in the art recognize that a number of plant cells or tissues are transformable in which after insertion of exogenous DNA and appropriate culture conditions the plant cells or tissues can form into a differentiated plant. Tissue suitable for these purposes can include immature embryos, scutellar tissue, suspension cell cultures, immature inflorescence, shoot meristem, nodal explants, callus tissue, hypocotyl tissue, cotyledons, roots, and leaves.

The regeneration, development, and cultivation of plants from transformed plant protoplast or explants is well taught in the art (Weissbach and Weissbach, Methods for Plant Molecular Biology, (Eds.), Academic Press, Inc., San Diego, CA, 1988; Horsch et al., Science, 227: 1229-1231, 1985). This regeneration and growth process typically includes the steps of selecting transformed cells and culturing those individualized cells through the usual stages of embryonic development through the rooted plantlet stage. Transgenic embryos and seeds are similarly regenerated. In this method, transformants are generally cultured in the presence of a selective media which selects for the successfully transformed cells_and induces the regeneration of plant shoots (Fraley et al., Proc. Natl. Acad. Sci. U.S.A., 80: 4803, 1983). These shoots are typically obtained within two to four months. The resulting transgenic rooted shoots are thereafter planted in an appropriate plant growth medium such as soil. Cells that survive the exposure to the selective agent, or cells that have been scored positive in a screening assay, may be cultured in media that supports regeneration of plants. The shoots are then transferred to an appropriate root-inducing medium containing the selective agent and an antibiotic to prevent bacterial growth. Many of the shoots will develop roots. These are then transplanted to soil or other media to allow the continued development of roots. The method, as outlined, will generally vary depending an the particular plant strain employed.

The regenerated transgenic plants are self-pollinated to provide homozygous transgenic plants. Alternatively, pollen obtained from the regenerated transgenic plants may be crossed with non-transgenic plants, preferably inbred lines of agronomically important species. Conversely, pollen from non-transgenic plants may be used to pollinate the regenerated transgenic plants.

The transgenic plant may pass along the transformed nucleic acid sequence to its progeny. The transgenic plant is preferably homozygous for the transformed nucleic acid sequence and transmits that sequence to all of its offspring upon as a result of sexual reproduction. Progeny may be grown from seeds produced by the transgenic plant. These additional plants may then be self-pollinated to generate a true breeding line of plants.

The progeny from these plants are evaluated, among other things, for gene expression.

The gene expression may be detected by several common methods such as western blotting, northern blotting, immunoprecipitation, and ELISA. The transformed plants are also analyzed for the presence of the genes of interest and the expression level and/or profile conferred by the non-coding regulatory elements of the present invention. Those of skill in the art are aware of the numerous methods available for the analysis of transformed plants. For example, methods for plant analysis include Southern blots or northern blots, PCR-based approaches, biochemical analyses, phenotypic screening methods, field evaluations, and immunodiagnostic assays. Methods for specifically transforming dicots are well known to those skilled in the art. Transformation and plant regeneration using these methods have been described for a number of crops including cotton *(Gossypium hirsutum),* soybean *(Glycine max),* peanut *(Arachis hypogaea),* and members of the genus *Brassica.* Methods for transforming dicots, primarily by use of *Agrobacterium tumefaciens* and obtaining transgenic plants have been published for cotton (U.S. Patent Nos. 5,004,863; 5,159,135; and 5,518,908); soybean (U.S. Patent Nos. 5,569,834; and 5,416,011; McCabe, et al., Biotechnolgy, 6: 923, 1988; Christou et al., Plant Physiol. 87:671-674 (1988)); *Brassica* (U.S. Patent No. 5,463,174); peanut (Cheng et al., Plant Cell Rep. 15:653-657 (1996), McKently et al., Plant Cell Rep. 14:699-703 (1995)); papaya; and pea (Grant et al., Plant Cell Rep. 15:254258 (1995)).

Methods for transforming monocots are well known to those skilled in the art.

Transformation and plant regeneration using these methods have been described for a number of crops including barley *(Hordeum vulgarae);* maize *(Zea mays);* oats *(Avena sativa);* orchard grass *(Dactylis glomerata);* rice *(Oryza sativa,* including indica and japonica varieties); sorghum *(Sorghum bicolor);* sugar cane *(Saccharum sp);* tall fescue *(Festuca arundinacea);* turfgrass species (e.g. species: *Agrostis stolonifera, Poa pratensis, Stenotaphrum secundatum);* wheat *(Triticum aestivum),* and alfalfa *(Medicago sativa).* It is apparent to those of skill in the art that a number of transformation methodologies can be used and modified for production of stable transgenic plants from any number of target crops of interest.

It is apparent to those of skill in the art that a number of transformation methodologies can be used and modified for production of stable transgenic plants from any number of target crops of interest.

The transformed plants are analyzed for the presence of the genes of interest and the expression level and/or profile conferred by the promoters of the present invention. Those of skill in the art are aware of the numerous methods available for the analysis of transformed plants. For example, methods for plant analysis include Southern blots or northern blots, PCR-based approaches, biochemical analyses, phenotypic screening methods, field evaluations, and immunodiagnostic assays.

### Transgenic Plants and Transgenic Seeds

The seeds of this invention can be harvested from fertile transgenic plants and be used to grow progeny generations of transformed plants including hybrid plant lines comprising the construct of this invention and expressing a gene of agronomic interest. Also described are parts of the plants. Plant parts, without limitation, include seed, endosperm, ovule and pollen. Preferably the plant part is a seed.

### Utilization of Transgenic Plants

A method of inhibiting weed growth in a field of transgenic crop plants comprising: (i) planting transgenic plants transformed with an expression cassette comprising (a) an ANT1-NCRE polynucleotide molecule active in the plant and operably linked to a DNA molecule encoding a glyphosate resistant EPSPS and (ii) applying glyphosate to the field at an application rate that inhibits the growth of weeds, wherein the growth and yield of the transgenic crop plant is not substantially affected by the glyphosate application. In particular the non-coding regulatory element is SEQ ID NO:1 or SEQ ID NO:2. The glyphosate application rate is the effective rate necessary to control weeds in a particular glyphosate tolerant crop, these rates may range from 0.56 to 17.9 kg/ha (8 oz/A to 256 oz/A), preferably 1.12 to 8.97 kg/ha (16 oz/A to 128 oz/A), more preferably 2.24 to 6.72 kg/ha (32 oz/A to 96 oz/A). The glyphosate is applied at least once during the growth of the glyphosate tolerant crop and may be applied 2, 3 or 4 times during the growth of the crop or more as necessary to control weeds in the field. In particular the transgenic plants are capable of tolerating an application rate up to 17.9 kg/ha (256 ounces/acre). In particular the transgenic plants are capable of tolerating an application rate ranging from 0.56 to 8.97 kg/ha (8 to 128 ounces/acre). In particular the transgenic plants are capable of tolerating an application rate ranging from 2.24 to 6.72 kg/ha (32 to 96 ounces/acre).

### Other Transformed Organisms

Any of the above described promoters and transcribable polynucleotide molecule sequences may be introduced into any cell or organism such as algae cell, algae, fungal cell, fungi, bacterial cell, or insect cell. Preferred hosts and transformants include: fungal cells such as *Aspergillus,* yeasts, insects, bacteria and algae.

The transformed cell or organism is preferably prokaryotic, more preferably a bacterial cell, even more preferably a *Agrobacterium, Bacillus, Escherichia, Pseudomonas* cell, and most preferably is an *Escherichia coli* cell. Alternatively, the transformed organism is preferably a yeast or fungal cell. The yeast cell is preferably a *Saccharomyces cerevisiae, Schizosaccharomyces pombe,* or *Pichia pastoris.*

Having now generally described the invention, the same will be more readily understood through reference to the following examples. Examples no longer covered by the claims are for illustrative purposes.

### EXAMPLES

### Example 1: Polynucleotide Molecule Identification and Cloning

A DNA molecule was isolated from an *Arabidopsis thaliana* Ant1 gene comprising a promoter, leader, and intron (referred to herein as P-At.Ant1, L-At.Ant1, and I-At.Ant1, respectively). Genomic DNA from *Arabidopsis thaliana* was isolated and then used in a DNA amplification method to isolate the At.Ant1 promoter, leader, and intron DNA molecule of the present invention. The DNA molecule was isolated from the genomic DNA template by a PCR based method using a High Fidelity PCR kit (Roche, Indianapolis, IN) that amplified the At.Ant1 5'untranslated region using the DNA primers Ant1-Hind3-For22 (SEQ ID NO:5) and Ant1-NcoI-Rev22 (SEQ ID NO:6). The PCR was set up in 2 X 50 4µl reactions as the following: dH₂O 80 µl; 10 mM dNTP 2µl; 10X buffer 10 µl; genomic DNA (50 ng) µl; Ant1-Hind3-For22 (10 µM) 3 µl; Ant1-NcoI-Rev22 (10 µM) 3µl; Enzyme 1 µl. PCR was carried out on a MJ Research PTC-200 thermal cycler (MJ Research, Waltham, MA) using the following program: Step 1 94°C for 3 min; Step 2 94°C for 20 s; Step 3 54°C for 20 s; Step 4 68°C for 20 s; Step 5 go to step 2, 30 times; Step 6 End. The PCR product was purified using QIAquick Gel Extraction kit (Qiagen Corp., Valencia, CA). The purified PCR product was digested with NcoI and HindIII and inserted by ligation into plasmid shuttle vector pMON70501. The isolated DNA molecule was sequenced using standard DNA sequencing methods.

The DNA sequence for the isolated DNA molecule that comprises the 5'untranslated regulatory elements of the At.Ant1 gene are shown in SEQ ID NO:1. The fragments of this molecule comprise the promoter element (P-At.Ant1) provided as SEQ ID NO: 2; the DNA sequence for the leader-intron element provided as SEQ ID NO:3; and the DNA sequence for the intron (I-At.Ant1) provided as SEQ ID NO: 4. In SEQ ID NO:3, the leader elements (L-At.Ant1) flank the intron element (nucleotide position 8986 to 9580).

A plant expression cassette was constructed to link the DNA molecule of SEQ ID NO:1 to the *Arabidopsis* EPSPS chloroplast transit peptide sequence, TS-At.ShkG-CTP2; the coding sequence for the glyphosate resistant EPSPS gene isolated from *Agrobacterium tumefaciens* strain CP4, *aroA:CP4* (glyphosate resistant EPSPS:CP4 protein, U.S. Patent 5,633,435); and the 3' nontranslated region of the ribulose bisphosphate carboxylase gene from *Pisum sativum,* T-Ps.RbcS2-E9. The expression cassette is flanked by the *Agrobacterium tumefaciens* right border (RB) and left border (LB) regions. The plasmid (pMON71524, Figure 1) was screened by restriction enzymes digestion and sequencing to confirm that the correct sequence was present.

### Example 2: Promoter Characterization in Arabidopsis thaliana

Each gene of interest may be amplified from a genomic or cDNA library using primers specific to sequences upstream and downstream of the coding region. Transformation vectors are prepared to constitutively transcribe DNA in either sense orientation (for enhanced protein expression) or anti-sense orientation (for endogenous gene suppression) under the control of an enhanced Cauliflower Mosaic Virus 35S promoter (U.S. patent 5,359,142) directly or indirectly (Moore et al. PNAS 95:376-381, 1998; Guyer et al. Genetics 149: 633-639, 1998; WO99/27119). The transformation vectors also contain a bar gene as a selectable marker for resistance to glufosinate herbicide. The transformation of Arabidopsis plants is carried out using the vacuum infiltration method known in the art (Bethtold et al.

Methods Mol. Biol. 82:259-66, 1998). Seeds harvested from the plants, named as R₁ seeds, are subsequently grown in a glufosinate-containing selective medium to select for plants which are actually transformed and which produced T2 transgenic seed.

Analysis of glyphosate herbicide tolerance DNA constructs in transgenic *Arabidopsis* was used as an screen to identify promoters that have a constitutive expression pattern and that are useful for herbicide tolerance in plants (for example, see the method described in WO02092856 for *Arabidopsis* transformation by vacuum infiltration and glyphosate treatment). Seeds from R₀ plants were harvested, sterilized and placed on 50 mM glyphosate agar media for selection of transformants. R₁ seedlings that germinated on glyphosate-containing agar were transferred to soil after 7 d.

Events from each construct were then analyzed for vegetative and reproductive tolerance to glyphosate. Plants transformed with pMON71524 (Figure 1) were compared to plants transformed with pMON26140 (Figure 2., a plasmid containing the plant expression cassette of a P-FMV.35S promoter operably linked to the petunia heat shock 70 leader (L-Ph.DnaK) and the *Arabidopsis* chloroplast transit peptide from the ShkG gene (TS-At.ShkG-CTP2) the aroA:CP4.nno artificial polynucleotide sequence encoding the EPSPS:CP4 protein). After three weeks plants in the rosette stage were sprayed with 0 kg/ha (0 oz/A (ounce per acre)), 1.68 kg/ha (24 oz/A), or 8.97 kg/ha (128 oz/A) of Roundup^{®} Ultra herbicide. Each herbicide treatment was done on at least 20 separate transgenic events per construct. Plants with vegetative damage were discarded. Plants treated with Roundup^{®} Ultra that were morphologically similar to non-treated plants and which also formed siliques filled with seeds were considered reproductively tolerant. Data are provided in Table 1 below. Measurements are provided as the percentage of fertile herbicide tolerant events out of the total events analyzed for each construct at each glyphosate dosage. The pMON26140 construct was shown to provide plants with 82% vegetative tolerance to Roundup^{®} Ultra at the 24 oz/A rate and 72% vegetative tolerance at the 8.97 kg/ha (128 oz/A) rate, but no reproductive tolerance was observed at these rates of Roundup^{®} Ultra treatment (0%, Table 1). The construct pMON71524 containing the At.Ant1 promoter and regulatory elements, provided plants with 100% vegetative glyphosate tolerance and a high level of reproductive tolerance, 73 percent of the pMON71524 plants were fertile when treated with 1.68 kg/ha (24 oz/A) Roundup® Ultra, and 92 percent were fertile when treated with 8.97 kg/ha (128 oz/A) Roundup^{®} Ultra. This result demonstrates the constitutive pattern of expression of the Ant1 regulatory elements and the ability of these elements to provide an agronomically useful phenotype.

**Table 1: Glyphosate reproductive tolerance in Arabidopsis**

| Construct | Percent Fertile Tolerant Events treated with 1.68 kg/ha (24 oz/A) Roundup^{®} | Percent Fertile Tolerant Events treated with 8.97 kg/ha (128 oz/A) Roundup^{®} |
|---|---|---|
| pMON26140 | 0% | 0% |
| PMON71524 | 73% | 92% |

### Example 3: Glyphosate tolerance in soybean

This example illustrates plant transformation useful in producing the transgenic soybean plants with constructs containing ANT1-NCRE molecules of this invention, and the resultant production and identification of transgenic seed for transgenic soybean having an improved agronomic trait, i.e. improved nitrogen use efficiency, improved yield, improved water use efficiency and/or improved growth under cold stress as compared to control plants.

For Agrobacterium-mediated transformation, soybean seeds are germinated overnight and the meristem explants excised. The meristems and the explants are placed in a wounding vessel. Soybean explants and induced Agrobacterium cells from a strain containing construct plasmid DNA with the gene of interest cassette and a plant selectable marker cassette are mixed no later than 14 h from the time of initiation of seed germination and wounded using sonication. Following wounding, explants are placed in co-culture for 2-5 d at which point they are transferred to selection media for 6-8 weeks to allow selection and growth of transgenic shoots. Trait positive shoots are harvested approximately 6-8 weeks post bombardment and placed into selective rooting media for 2-3 weeks. Shoots producing roots are transferred to the greenhouse and potted in soil. Shoots that remain healthy on selection, but do not produce roots are transferred to non-selective rooting media for an additional two weeks. Roots from any shoots that produce roots off selection are tested for expression of the plant selectable marker before they are transferred to the greenhouse and potted in soil.

Transgenic soybean seed and plants with recombinant DNA from the constructs containing ANT1-NCRE molecules are prepared by a transformation method such as described above. The transgenic seed, plantlets and progeny plants are screened for the presence of a desirable agronomic trait, for example, nitrogen use efficiency, yield, water use efficiency, and cold tolerance, to identify transgenic soybean seed for transgenic soybean plants having an improved agronomic trait, i.e. improved nitrogen use efficiency, improved yield, improved water use efficiency and/or improved growth under cold stress as compared to control plants.

The DNA constructs, pMON71524 and pMON20999 (Figure 3, containing P-FMV.35S promoter operably linked to the *aroA:CP4* transgene, the plant expression cassette of this plasmid is essentially identical to pMON26140 that was used in the *Arabidopsis* experiments) were used to transform soybean *(Glycine max)* by an *Agrobacterium* mediated method (for example, see U.S. Patent Nos. 6,384,301, 5,569,834, and 5,416,011).

R₁ soybean plants were tested for vegetative tolerance to glyphosate. For the R₁ evaluations, typically 48 seeds per event were planted and an ELISA analysis was performed to identify the positive transformants and to determine the segregation ratio. Plants were sprayed typically sprayed with approximately 3.64 kg/ha (52 oz/A) of Roundup UltraMax^{®} herbicide at the V1 stage. Approximately one week post-spray the events were evaluated for chlorosis. Copy number and zygosity were also assessed. Other observations taken included: emergence, segregation, pod set (timing of), plant height, and maturity. Those events that had one copy of the transgene, no vector backbone, and vegetative glyphosate tolerance were advanced to the R₂ nursery to evaluate reproductive tolerance, these events are referred to as the R₂ events. Plants from the R₂ events were tested for reproductive tolerance to glyphosate. Plants treated with Roundup UltraMax^{®} that were morphologically similar to non-treated plants and which also produced seeds were considered tolerant. Data showing enhanced reproductive tolerance are provided in Table 2.

**Table 2: Glyphosate tolerance in Soybean (3.64 kg/ha (52 oz/A) Roundup UltraMax^{®})**

| Construct | Vegetative Tolerance Percent R₁ events | Reproductive Tolerance Percent R₂ events |
|---|---|---|
| pMON71524 | 67% | 64% |
| pMON20999 | 55% | 0% |

Events containing the *aroA:CP4* transgene operably linked to the *Arabidopsis* Ant promoter, leader, and intron (SEQ ID NO:1) showed good vegetative and reproductive tolerance to glyphosate in soybean when compared to the control. Sixty seven percent of the R₁ events generated were found to have vegetative tolerance and sixty four percent of the R₂ events showed reproductive tolerance in soybean to glyphosate in the R₂ generation.

The promoter and regulatory elements of the present invention can be linked to other known DNA molecules that encode glyphosate resistant EPSPS enzymes. These other enzymes include microbial class II EPSP synthases and modified dass I EPSP synthases, for example, the modified maize EPSPS gene encoding an EPSPS protein having isoleucine at position 102 and serine at position 106 (U.S. Patent 6,040,497). The genes that encode enzymes that degrade or inactivate glyphosate are also contemplated to be useful to confer plant tolerance to glyphosate when operably linked to the Ant1 gene 5' regulatory elements, these include, for example, the glyphosate oxidoreductase and glyphosate-N-acetyl transferase (GOX, U.S. Patent 5,463,175 and GAT, U.S. Patent publication 20030083480).
## Claims

1. A polynucleotide molecule having gene regulatory activity consisting of an adenylate translocase gene non-coding regulatory element polynucleotide molecule, that
(a) exhibits an 95% or greater identity to the full-length sequence of SEQ ID NO:1, or to a sequence that exhibits complete complementarity thereto, or any fragment of SEQ ID NO:1 comprising at least 95 contiguous nucleotide bases, each thereof capable of directing transgene expression in glyphosate sensitive reproductive organs; or
(b) has the sequence of SEQ ID NO:1;
wherein said polynucleotide molecule is operably linked to a transcribable polynucleotide molecule that is heterologous to said polynucleotide molecule.

2. The polynucleotide molecule of claim 1 which is SEQ ID NO:1 or SEQ ID NO:2.

3. A polynucleotide construct comprising a polynucleotide molecule comprising an adenylate translocase gene non-coding regulatory element polynucleotide molecule, that
(a) exhibits an 95% or greater identity to the full-length sequence of SEQ ID NO:1, or to a sequence that exhibits complete complementarity thereto, or any fragment of SEQ ID NO:1 comprising at least 95 contiguous nucleotide bases, each thereof capable of directing transgene expression in glyphosate sensitive reproductive organs; or
(b) has the sequence of SEQ ID. NO:1;
wherein said polynucleotide molecule is operably linked to a transcribable polynucleotide molecule that is heterologous to said polynucleotide molecule.

4. The polynucleotide construct of claim 3, wherein the polynucleotide molecule comprises the nucleic acid sequence of SEQ ID NO:1 or SEQ ID NO:2.

5. The polynucleotide construct of claim 3, wherein said transcribable polynucleotide molecule is a gene of agronomic interest.

6. The polynucleotide construct of claim 3, wherein said transcribable polynucleotide molecule is a herbicide tolerance gene.

7. The polynucleotide construct of claim 6, wherein said herbicide tolerance gene is selected from genes that encode for phosphinothricin acetyltransferase, glyphosate resistant EPSPS, hydroxyphenyl pyruvate dehydrogenase, dalapon dehalogenase, bromoxynil resistant nitrilase, anthranilate synthase, glyphosate oxidoreductase and glyphosate-N-acetyl transferase.

8. A transgenic plant cell having the polynucleotide construct of claim 3 integrated into the genome.

9. A transgenic plant having the polynucleotide construct of claim 3 integrated into the genome.

10. The transgenic plant of claim 9, comprising an polynucleotide molecule of SEQ ID NO:1, wherein said polynucleotide molecule is operably linked to a transcribable polynucleotide molecule which in turn is operably linked to a 3' transcription termination nucleic acid molecule.

11. The transgenic plant of claim 9 or 10, wherein said plant
(i) is a monocotyledonous plant selected from wheat, maize, rye, rice, oat, barley, turfgrass, sorghum, millet and sugarcane; or
(ii) is a dicotyledonous plant selected from tobacco, tomato, potato, soybean, cotton, canola, sunflower and alfalfa.

12. A seed of said transgenic plant of claim 11 having the polynucleotide construct of claim 3 integrated into the genome.

13. A method of inhibiting weed growth in a field of transgenic glyphosate tolerant crop plants comprising:
(i) planting the transgenic plants transformed with an expression cassette comprising a polynucleotide molecule having gene regulatory activity comprising an adenylate translocase gene non-coding regulatory element polynucleotide molecule, that
(a) exhibits an 95% or greater identity to the full-length sequence of SEQ ID NO:1, or to a sequence that exhibits complete complementarity thereto, or any fragment of SEQ ID NO:1 comprising at least 95 contiguous nucleotide bases, each thereof capable of directing transgene expression in glyphosate sensitive reproductive organs; or
(b) has the sequence of SEQ ID NO:1,
operably linked to a polynucleotide molecule encoding a glyphosate tolerance gene and
(ii) applying glyphosate to the field at an application rate that inhibits the growth of weeds, wherein the growth and yield of the transgenic crop plant is not substantially affected by the glyphosate application.

14. The method of claim 13, wherein the polynucleotide molecule comprises the nucleic acid sequence of SEQ ID NO:1 or SEQ ID NO:2.

15. The method of claims 13 or 14, wherein said glyphosate tolerance gene is selected from a gene encoding for a glyphosate resistant EPSPS, a glyphosate oxidoreductase and a glyphosate-N-acetyl transferase.

16. The method of claims 13 or 14, wherein the transgenic plants are capable of tolerating an application rate ranging up to 17.9 kg/ha (256 ounces/acre).

17. The method of claims 13 or 14, wherein the application of glyphosate is at least once during the growth of the crop.

## Patentansprüche

1. Polynucleotidmolekül mit Gen-regulatorischer Aktivität, das aus einem Polynucleotidmolekül in Form eines nichtcodierenden regulatorischen Elements eines Adenylat-Translocase-Gens besteht, welches
(a) eine Identität von 95% oder mehr mit der vollen Länge der Sequenz von SEQ ID Nr. 1 oder mit einer Sequenz, die völlige Komplementarität dazu aufweist, oder mit irgendeinem Fragment von SEQ ID Nr. 1, das wenigstens 95 aufeinanderfolgende Nucleotidbasen umfasst, aufweist, wobei jedes davon die Transgen-Expression in glyphosatempfindlichen Reproduktionsorganen lenken kann; oder
(b) die Sequenz von SEQ ID Nr. 1 aufweist;
wobei das Polynucleotidmolekül funktionell mit einem transkribierbaren Polynucleotidmolekül, das heterolog zu dem Polynucleotidmolekül ist, verknüpft ist.

2. Polynucleotidmolekül gemäß Anspruch 1, bei dem es sich um SEQ ID Nr. 1 oder SEQ ID Nr. 2 handelt.

3. Polynucleotidkonstrukt, welches ein Polynucleotidmolekül umfasst, das aus einem Polynucleotidmolekül in Form eines nichtcodierenden regulatorischen Elements eines Adenylat-Translocase-Gens besteht, welches
(a) eine Identität von 95% oder mehr mit der vollen Länge der Sequenz von SEQ ID Nr. 1 oder mit einer Sequenz, die völlige Komplementarität dazu aufweist, oder mit irgendeinem Fragment von SEQ ID Nr. 1, das wenigstens 95 aufeinanderfolgende Nucleotidbasen umfasst, aufweist, wobei jedes davon die Transgen-Expression in glyphosatempfindlichen Reproduktionsorganen lenken kann; oder
(b) die Sequenz von SEQ ID Nr. 1 aufweist;
wobei das Polynucleotidmolekül funktionell mit einem transkribierbaren Polynucleotidmolekül, das heterolog zu dem Polynucleotidmolekül ist, verknüpft ist.

4. Polynucleotidkonstrukt gemäß Anspruch 3, wobei das Polynucleotidmolekül die Nucleinsäuresequenz von SEQ ID Nr. 1 oder SEQ ID Nr. 2 umfasst.

5. Polynucleotidkonstrukt gemäß Anspruch 3, wobei das transkribierbare Polynucleotidmolekül ein Gen von agronomischem Interesse ist.

6. Polynucleotidkonstrukt gemäß Anspruch 3, wobei das transkribierbare Polynucleotidmolekül ein Herbizidtoleranz-Gen ist.

7. Polynucleotidkonstrukt gemäß Anspruch 6, wobei das Herbizidtoleranz-Gen aus Genen ausgewählt ist, die für Phosphinothricin-Acetyltransferase, Glyphosat-resistentes EPSPS, Hydroxyphenylpyruvat-Dehydrogenase, Dalapon-Dehalogenase, Bromoxynil-resistente Nitrilase, Anthranilat-Synthase, Glyphosat-Oxidoreductase und Glyphosat-N-Acetyltransferase codieren.

8. Transgene Pflanzenzelle, die das Polynucleotidkonstrukt gemäß Anspruch 3 in ihr Genom integriert hat.

9. Transgene Pflanze, die das Polynucleotidkonstrukt gemäß Anspruch 3 in ihr Genom integriert hat.

10. Transgene Pflanze gemäß Anspruch 9, die ein Polynucleotidmolekül gemäß SEQ ID Nr. 1 umfasst, wobei das Polynucleotidmolekül funktionell mit einem transkribierbaren Polynucleotidmolekül verknüpft ist, das wiederum funktionell mit einem 3'-Transkriptionsterminationsnucleinsäuremolekül verknüpft ist.

11. Transgene Pflanze gemäß Anspruch 9 oder 10, wobei die Pflanze
(i) eine einkeimblättrige Pflanze ist, die aus Weizen, Mais, Roggen, Reis, Hafer, Gerste, Rasengras, Sorghum, Hirse und Zuckerrohr ausgewählt ist; oder
(ii) eine zweikeimblättrige Pflanze ist, die aus Tabak, Tomate, Kartoffel, Sojabohne, Baumwolle, Canola, Sonnenblume und Luzerne ausgewählt ist.

12. Samen der transgenen Pflanze gemäß Anspruch 11, der das Polynucleotidkonstrukt gemäß Anspruch 3 in sein Genom integriert hat.

13. Verfahren zur Hemmung des Unkrautwachstums in einem Feld von transgenen glyphosattoleranten Kulturpflanzen, umfassend:
das Einpflanzen der transgenen Pflanzen, die mit einer Expressionscassette transformiert sind, welche ein Polynucleotidmolekül umfasst, das Genregulatorische Aktivität aufweist und ein Polynucleotidmolekül in Form eines nichtcodierenden regulatorischen Elements eines Adenylat-Translocase-Gens umfasst, welches
(a) eine Identität von 95% oder mehr mit der vollen Länge der Sequenz von SEQ ID Nr. 1 oder mit einer Sequenz, die völlige Komplementarität dazu aufweist, oder mit irgendeinem Fragment von SEQ ID Nr. 1, das wenigstens 95 aufeinanderfolgende Nucleotidbasen umfasst, aufweist, wobei jedes davon die Transgen-Expression in glyphosatempfindlichen Reproduktionsorganen lenken kann; oder
(b) die Sequenz von SEQ ID Nr. 1 aufweist;
funktionell mit einem Polynucleotidmolekül, das ein Glyphosat-Toleranz-Gen codiert, verknüpft ist; und
(ii) das Aufbringen von Glyphosat auf das Feld mit einer Auftragungsmenge, die das Wachstum von Unkräutern hemmt, wobei das Wachstum und der Ertrag der transgenen Kulturpflanze durch die Glyphosat-Anwendung nicht wesentlich beeinflusst wird.

14. Verfahren gemäß Anspruch 13, wobei das Polynucleotidmolekül die Nucleinsäuresequenz von SEQ ID Nr. 1 oder SEQ ID Nr. 2 umfasst.

15. Verfahren gemäß Anspruch 13 oder 14, wobei das Glyphosat-Toleranz-Gen aus einem Gen ausgewählt ist, das für Glyphosat-resistentes EPSPS, eine Glyphosat-Oxidoreductase und eine Glyphosat-N-Acetyltransferase codiert.

16. Verfahren gemäß Anspruch 13 oder 14, wobei die transgenen Pflanzen eine Auftragsmenge im Bereich von bis zu 17,9 kg/ha (256 Unzen/Morgen) tolerieren können.

17. Verfahren gemäß Anspruch 13 oder 14, wobei die Auftragung des Glyphosats wenigstens einmal während des Wachstums der Kulturpflanze erfolgt.

## Revendications

1. Molécule de polynucléotide dotée d'une activité régulatrice de gènes constituée d'une molécule de polynucléotide d'élément régulateur non codant du gène de l'adénylate translocase, qui
(a) présente une identité de 95 % ou supérieure avec la séquence pleine longueur de SEQ ID NO : 1, ou avec une séquence qui présente une complémentarité complète avec celle-ci, ou tout fragment de SEQ ID NO : 1 comprenant au moins 95 bases nucléotidiques contiguës, chacune d'entre elles capable de diriger l'expression de transgènes dans des organes reproducteurs sensibles au glyphosate ; ou
(b) a la séquence de SEQ ID NO : 1 ;
dans laquelle ladite molécule de polynucléotide est liée de façon fonctionnelle à une molécule de polynucléotide pouvant être transcrite qui est hétérologue à ladite molécule de polynucléotide.

2. Molécule de polynucléotide selon la revendication 1 qui est SEQ ID NO : 1 ou SEQ ID NO : 2.

3. Construction polynucléotidique comprenant une molécule de polynucléotide comprenant une molécule de polynucléotide d'élément régulateur non codant du gène de l'adénylate translocase, qui
(a) présente une identité de 95 % ou supérieure avec la séquence pleine longueur de SEQ ID NO : 1, ou avec une séquence qui présente une complémentarité complète avec celle-ci, ou tout fragment de SEQ ID NO : 1 comprenant au moins 95 bases nucléotidiques contiguës, chacune d'entre elles capable de diriger l'expression de transgènes dans des organes reproducteurs sensibles au glyphosate ; ou
(b) a la séquence de SEQ ID NO : 1 ;
dans laquelle ladite molécule de polynucléotide est liée de façon fonctionnelle à une molécule de polynucléotide pouvant être transcrite qui est hétérologue à ladite molécule de polynucléotide.

4. Construction polynucléotidique selon la revendication 3, dans laquelle la molécule de polynucléotide comprend la séquence d'acide nucléique de SEQ ID NO : 1 ou SEQ ID NO : 2.

5. Construction polynucléotidique selon la revendication 3, dans laquelle ladite molécule de polynucléotide pouvant être transcrite est un gène d'intérêt agronome.

6. Construction polynucléotidique selon la revendication 3, dans laquelle ladite molécule de polynucléotide pouvant être transcrite est un gène de tolérance aux herbicides.

7. Construction polynucléotidique selon la revendication 6, dans laquelle ledit gène de tolérance aux herbicides est choisi parmi des gènes qui codent pour la phosphinothricine acétyltransférase, l'EPSPS résistante au glyphosate, l'hydroxyphénylpyruvate déshydrogénase, la dalapon déshalogénase, la nitrilase résistante au bromoxynil, l'anthranylate synthase, la glyphosate oxydoréductase et la glyphosate-N-acétyltransférase.

8. Cellule de plante transgénique ayant la construction polynucléotidique selon la revendication 3 intégrée dans le génome.

9. Plante transgénique ayant la construction polynucléotidique selon la revendication 3 intégrée dans le génome.

10. Plante transgénique selon la revendication 9, comprenant une molécule de polynucléotide de SEQ ID NO : 1, dans laquelle ladite molécule de polynucléotide est liée de façon fonctionnelle à une molécule de polynucléotide pouvant être transcrite qui à son tour est liée de façon fonctionnelle à une molécule d'acide nucléique de terminaison de la transcription en 3'.

11. Plante transgénique selon la revendication 9 ou 10, dans laquelle ladite plante
(i) est une plante monocotylédone choisie parmi le blé, le maïs, le seigle, le riz, l'avoine, l'orge, le gazon, le sorgho, le millet et la canne à sucre ; ou
(ii) est une plante dicotylédone choisie parmi le tabac, la tomate, la pomme de terre, le soja, le coton, le colza, le tournesol et la luzerne.

12. Graine de ladite plante transgénique selon la revendication 11 ayant la construction polynucléotidique selon la revendication 3 intégrée dans le génome.

13. Procédé d'inhibition de la croissance des mauvaises heures dans un champ de plantes transgéniques de culture tolérantes au glyphosate comprenant :
(i) la mise en terre des plantes transgéniques transformées avec une cassette d'expression comprenant une molécule de polynucléotide dotée d'une activité régulatrice de gènes comprenant une molécule de polynucléotide d'élément régulateur non codant du gène de l'adénylate translocase, qui
(a) présente une identité de 95 % ou supérieure avec la séquence pleine longueur de SEQ ID NO : 1, ou avec une séquence qui présente une complémentarité complète avec celle-ci, ou tout fragment de SEQ ID NO : 1 comprenant au moins 95 bases nucléotidiques contiguës, chacune d'entre elles capable de diriger l'expression de transgènes dans des organes reproducteurs sensibles au glyphosate ; ou
(b) a la séquence de SEQ ID NO : 1,
liée de façon fonctionnelle à une molécule de polynucléotide codant pour un gène de tolérance au glyphosate et
(ii) l'application de glyphosate sur le champ à un taux d'application qui inhibe la croissance des mauvaises herbes, dans lequel la croissance et le rendement de la plante transgénique de culture ne sont pas sensiblement affectés par l'application de glyphosate.

14. Procédé selon la revendication 13, dans lequel la molécule de polynucléotide comprend la séquence d'acide nucléique de SEQ ID NO : 1 ou SEQ ID NO : 2.

15. Procédé selon les revendications 13 ou 14, dans lequel ledit gène de tolérance au glyphosate est choisi parmi un gène codant pour une EPSPS résistante au glyphosate, une glyphosate oxydoréductase et une glyphosate-N-acétyltransférase.

16. Procédé selon les revendications 13 ou 14, dans lequel les plantes transgéniques sont capables de tolérer un taux d'application situé dans la plage jusqu'à 17,9 kg/ha (256 onces/acre).

17. Procédé selon les revendications 13 ou 14, dans lequel l'application de glyphosate est au moins une fois durant la croissance de la récolte.
